# EUROPÄISCHE PATENTSCHRIFT

(11) **EP 3 132 043 B1**
(45) Veröffentlichungstag und Bekanntmachung des Hinweises auf die Patenterteilung: **06.03.2019**
(21) Anmeldenummer: 15715750.4
(22) Anmeldetag: 15.04.2015
(51) Int. Cl.: C12N 15/861

(54) **VIRALER VEKTOR FÜR DEN ZIELGERICHTETEN GENTRANSFER IN GEHIRN UND RÜCKENMARK**
VIRAL VECTOR FOR THE TARGETED TRANSFER OF GENES IN THE BRAIN AND SPINAL CORD
VECTEUR VIRAL POUR LE TRANSFERT GÉNIQUE CIBLÉ DANS LE CERVEAU ET LA MOELLE ÉPINIÈRE

(30) Priorität: 17.04.2014 DE 102014207498
(43) Veröffentlichungstag der Anmeldung: 22.02.2017
(60) Teilanmeldung: 19153559.0
(73) Patentinhaber: Boehringer Ingelheim International GmbH, 55216 Ingelheim am Rhein (DE)
(72) Erfinder: KÖRBELIN, Jakob, 22769 Hamburg (DE); MICHELFELDER, Stefan, 79211 Denzlingen (DE); TREPEL, Martin, 22149 Hamburg (DE)
(74) Vertreter: Uexküll & Stolberg
(86) Internationale Anmeldenummer: PCT/EP2015/058123
(87) Internationale Veröffentlichungsnummer: WO 2015/158749

(56) Entgegenhaltungen:
- WO-A1-2010/127097
- DATABASE EMBL [Online] 29. Dezember 2008 (2008-12-29), "Talaromyces stipitatus ATCC 10500 phenylacetyl-CoA ligase, putative", XP002740760, gefunden im EBI accession no. EMBL:EED12934 Database accession no. EED12934
- BENYHE SANDOR ET AL: "Met-5-enkephalin-Arg-6-Phe-7, an endogenous neuropeptide, binds to multiple opioid and nonopioid sites in rat brain", JOURNAL OF NEUROSCIENCE RESEARCH, Bd. 48, Nr. 3, 1997, Seiten 249-258, XP002740763, ISSN: 0360-4012
- MEUNIER J-C ET AL: "ISOLATION AND STRUCTURE OF THE ENDOGENOUS AGONIST OF OPIOID RECEPTOR-LIKE ORL1 RECEPTOR", NATURE, Bd. 377, 12. Oktober 1995 (1995-10-12), Seiten 532-535, XP002020240, NATURE PUBLISHING GROUP, UNITED KINGDOM ISSN: 0028-0836, DOI: 10.1038/377532A0
- ALLARD M ET AL: "Characterization of rat spinal cord receptors to FLFQPQRFamide, a mammalian morphine modulating peptide: a binding study", BRAIN RESEARCH, Bd. 500, Nr. 1-2, 23. Oktober 1989 (1989-10-23), Seiten 169-176, XP024273370, ELSEVIER, AMSTERDAM, NL ISSN: 0006-8993, DOI: 10.1016/0006-8993(89)90311-9 [gefunden am 1989-10-23]
- IGWE O J ET AL: "SPECIFIC BINDING OF SUBSTANCE P AMINO-TERMINAL HEPTAPEPTIDE SP 1-7 TO MOUSE BRAIN AND SPINAL CORD MEMBRANES", JOURNAL OF NEUROSCIENCE, Bd. 10, Nr. 11, 1990, Seiten 3653-3663, XP002740761, ISSN: 0270-6474
- BARTLETT J S ET AL: "SELECTIVE AND RAPID UPTAKE OF ADENO-ASSOCIATED VIRUS TYPE 2 IN BRAIN", HUMAN GENE THERAPY, Bd. 9, Nr. 8, 20. Mai 1998 (1998-05-20), Seiten 1181-1186, XP000857504, MARY ANN LIEBERT, NEW YORK ,NY, US ISSN: 1043-0342

## Beschreibung

Die Erfindung betrifft neuartige Kapsid-Proteine, die spezifisch an Zellen des Gehirns und/oder des Rückenmarks binden. Die Proteine können Bestandteil eines viralen Kapsids sein und dazu verwendet werden, einen rekombinanten viralen Vektor nach systemischer Verabreichung an ein Subjekt selektiv zum Gehirn und/oder zum Rückenmark zu leiten und dort für eine gewebespezifische Expression von einem oder mehreren Transgenen zu sorgen. Die Erfindung betrifft somit auch einen rekombinanten viralen Vektor, vorzugsweise einen AAV-Vektor, der ein erfindungsgemäßes Kapsid sowie mindestens ein in dem Kapsid verpacktes Transgen umfasst. Der virale Vektor eignet sich insbesondere zur therapeutischen Behandlung einer Erkrankung oder Funktionsstörung des Gehirns und/oder des Rückenmarks. Die Erfindung betrifft ferner Zellen und pharmazeutische Zusammensetzungen, die den erfindungsgemäßen viralen Vektor umfassen.

### HINTERGRUND DER ERFINDUNG

Die gentherapeutische Behandlung mittels viraler Vektoren stellt eine vielversprechende Behandlungsmöglichkeit für Erkrankungen dar, die auf eine konventionelle Behandlung nicht oder nicht in ausreichendem Maße ansprechen. Dieser Ansatz beruht auf der Einschleusung von therapeutischen Genen in den zu behandelnden Organismus mit Hilfe von Viren, die so modifizierte wurden, dass sie die Sequenz des entsprechenden Gens in ihrem Genom aufweisen. Virale Vektoren, die im Rahmen der Gentherapie bereits für gentherapeutische Therapieansätze verwendet wurden, basieren auf Retroviren, Lentiviren, Adenoviren und Adeno-assoziierte Viren.

Adeno-assozierte Viren (AAV) stellen vielversprechende Kandidaten für den Einsatz in der der klinischen Praxis dar, da sie als verhältnismäßig sicher eingestuft werden. AAV-Vektoren sind in der Lage, ein Transgen in ein Gewebe einzuschleusen und dort stabil und effizient zu exprimieren. Gleichzeitig weisen diese Vektoren keine bekannten Pathogenitätsmechanismen auf [1]. Besondere Bedeutung für eine klinische Anwendung kommt den AAV-Vektoren vom Serotyp 2 (AAV2) zu, die als besonders gut untersucht gelten. Nach Einschleusung durch AAV-Vektoren können die Transgene in verschiedenen Formen in der transfizierten Zelle vorliegen, z.B. als episomale, einzel- oder doppelsträngige DNA. Auch konkatamere Formen der DNA wurden in transduzierten Zellen nachgewiesen.

Das Genom von AAV2 wird von einem linearen, einzelsträngigen DNA-Molekül von etwa 4700 Nukleotiden Länge gebildet und umfasst an beiden Enden Inverted Terminal Repeats (ITRs). Das Genom umfasst ferner zwei große offene Leserahmen, die als Replikations-Region (rep) und Kapsid-Region (cap) bezeichnet werden. Die Replikations-Region kodiert für Proteine, die im Rahmen der Virusreplikation erforderlich sind. Die Kapsid-Region hingegen kodiert für die strukturellen Proteine VP1, VP2 und VP3, aus denen sich das ikosaedrische Kapsid des Virus zusammensetzt.

Wie die meisten im Stand der Technik bekannten, gentherapeutisch verwendbaren Vektoren allerdings weisen Wildtyp-AAV-Vektoren, wie z.B. die beschriebenen AAV2-Vektoren, keine ausreichende Spezifität für ein bestimmtes Gewebe auf, sondern infizieren eine große Bandbreite von Zelltypen. Bei systemischer Gabe dieser Wildtyp-Vektoren kommt es somit zu einer unzureichenden Transduktion der Zielgewebe, während aufgrund der unerwünschten Transduktion von anderen Geweben mit starken Immunreaktionen im behandelten Patienten gerechnet werden muss. Fortschritte bei der Entwicklung von viralen Vektoren, die eine erhöhte Spezifität für bestimmte Organe aufweisen, wurden in der Vergangenheit durch den Einsatz von Peptidliganden erreicht, die in der Lage sind, die Vektoren zu einem bestimmten Organ zu leiten [2-3]. Es konnte gezeigt werden, dass bestimmte Peptidliganden für ein "Homing" zu verschiedenen Organen wie z.B. zum Gehirn sorgen.

Literaturstelle [4] beschreibt ein Verfahren, welches das Screening nach Kapsiden von AAV2 mit modifiziertem Tropismus in randomisierten Peptidbanken erlaubt. Aus diesen Bibliotheken können Vektoren isoliert werden, die *in vitro* spezifisch einen gewünschten Zelltyp transduzieren. Jedoch wurde festgestellt, dass die auf diese Weise selektierten Kapside oftmals für eine Verwendung *in vivo* ungeeignet sind, da ihnen im Tiermodell die nötige Spezifität fehlte [5].

Das Gehirn stellt ein klinisch extrem relevantes Organ dar, da es Ausgangspunkt für eine Reihe von neurologischen Erkrankungen ist. Es wurden erhebliche Anstrengungen unternommen, dieses Organ für gentherapeutische Interventionen erreichbar zu machen [6-12]. Allerdings stellt die Blut-Hirn-Schranke, die gemeinschaftlich von Endothelzellen, Perizyten und Astrozyten gebildet wird und das Gehirn von zirkulierenden Partikeln, Toxinen und Botenstoffen abschottet, ein für gängige Vektorsysteme unüberwindbares Hindernis dar. Da bislang keine geeigneten Vektorsysteme verfügbar sind, die nach intravenöser Applikation zielgerichtet mit ausreichender Effizienz das Gehirn transduzieren, werden heutige Gentherapievektoren meist direkt in das Gehirn injiziert [13], was mit einem hohen Risiko für den Patienten behaftet ist. Die Möglichkeit, die Blut-Hirn-Schranke kurzzeitig für größere Partikel passierbar zu machen, z.B. durch Ultraschall [14] oder mittels chemischer Komponenten [7], wird ebenfalls als sehr riskant erachtet.

Es besteht daher ein großes Bedürfnis nach Mitteln, die in der Lage sind, den Tropismus viraler Vektoren zu modulieren und damit für eine ausreichende Zell- oder Gewebespezifität sorgen, die einen gezielten Transport eines viralen Vektors zu Geweben des Gehirn und Rückenmarks, ermöglicht. Solche Vektoren können für eine spezifische Expression von therapeutischen Genen in diesen Geweben sorgen, um auf diese Weise Erkrankungen und/oder Funktionsstörungen des Gehirns und Rückenmarks wirksam zu behandeln.

Die vorliegende Erfindung stellt virale Vektoren für den zielgerichteten Gentransfer in das Gehirn und Rückenmarks bereit. Die erfindungsgemäßen viralen Vektoren exprimieren auf ihrer Kapsid-Oberfläche bislang unbekannte Aminosäuresequenzen, die *in vivo* spezifisch von Rezeptoren auf den Neuronen des Gehirns und den Endothelzellen der Blutgefäße von Gehirn und Rückenmark erkannt werden. Somit transduzieren die viralen Vektoren der vorliegenden Erfindung nach systemischer Verabreichung an ein Subjekt spezifisch dessen Gewebe in Gehirn und Rückenmark.

Die erfindungsgemäßen viralen Vektoren ermöglichen darüber hinaus eine starke und lang anhaltende Expression eines Transgens in den Neuronen des Gehirns und den endothelialen Zellen der Blutgefäße von Gehirn und Rückenmark. Daher sind die Vektoren insbesondere für die gentherapeutische Behandlung von bestimmten Erkrankungen und Funktionsstörungen von Gehirn und Rückenmark geeignet. Es wurde darüber hinaus festgestellt, dass die die AAV-Vektoren nach der Transfektion lediglich eine geringfügige Immunreaktion in dem Wirt hervorrufen und somit besonders für die Gentherapie geeignet sind.

### BESCHREIBUNG DER ERFINDUNG

Im Rahmen der vorliegenden Erfindung wurden durch Selektion in einer randomisierten AAV2-Heptamer-Pepidbibliothek verschiedene für das Zentralnervensystem, d.h. für Gehirn und Rückenmark, spezifische Sequenzen identifiziert. Ein Heptamer, das eine besondere Spezifität für das Gehirn aufwies, war das Peptid NRGTEWD (SEQ ID NO:1). Auf Basis dieser Sequenz wurde der rekombinante virale Vektor rAAV2-NRGTEWD hergestellt, bei dem die Peptidsequenz NRGTEWD als Teilsequenz des Kapsid-Proteins VP1 exprimiert wird. Anschließend wurde der Vektor rAAV2- NRGTEWD intravenös an Mäuse verabreicht, wobei sowohl *in vitro* als auch *in vivo* eine eindeutige Spezifität des Vektors für das Zentralnervensystem beobachtet werden konnte. Sowohl Neuronen des Gehirns als auch Endothelzellen der Blutgefäße von Gehirn und Rückenmark wurden von dem Vektor transfiziert. Die Spezifität konnte durch Färbung mit CD31 nachgewiesen werden, wie es in den vorliegenden Beispielen beschrieben wird.

Eine weitere Gruppe von Peptiden, die ebenfalls eine Spezifität für Gehirn und Rückenmark zeigten, umfasste die Peptide ADGVQWT (SEQ ID NO:2), DDGVSWK (SEQ ID NO:3), SDGLTWS (SEQ ID NO:4) und SDGLAWV (SEQ ID NO:5). Diese Peptide enthielten jeweils das allgemeine Motiv XDGXXWX (SEQ ID NO:6).

Die vorliegende Erfindung stellt daher verschiedene für das Gehirn und das Rückenmark spezifische Kapsid-Proteine bereit, die in besonderer Weise dazu geeignet sind, therapeutische Mittel, wie z.B. virale Vektoren, gezielt in das Gehirn bzw. das Rückenmark eines zu behandelnden Subjekts zu leiten.

In einem ersten Aspekt betrifft die vorliegende Erfindung demgemäß ein Kapsid-Protein eines viralen Vektors, das spezifisch an Zellen des Gehirns und/oder des Rückenmarks bindet, wobei das Kapsid-Protein die Aminosäuresequenz von SEQ ID NO:1 oder eine Variante davon umfasst, wobei sich die Variante von der Aminosäuresequenz von SEQ ID NO:1 durch Modifikation von maximal einer Aminosäure unterscheidet. Das erfindungsgemäße Kapsid-Protein bindet vorzugsweise an Endothelzellen und/oder an Neuronen des Gehirns oder Rückenmarks.

Offenbart wird vorliegend ferner ein Peptid, Polypeptid oder Protein, das spezifisch an Zellen des Gehirns und/oder des Rückenmarks bindet, wobei das Peptid, Polypeptid oder Protein die allgemeine Aminosäuresequenz von SEQ ID NO:6 umfasst. Das Peptid, Polypeptid oder Protein kann eine der Sequenzen ADGVQWT (SEQ ID NO:2), DDGVSWK (SEQ ID NO:3), SDGLTWS (SEQ ID NO:4) oder SDGLAWV (SEQ ID NO:5) oder eine Variante derselben umfassen, wobei sich die Variante von der jeweiligen Aminosäuresequenz von SEQ ID NO:2-5 durch Modifikation von maximal einer Aminosäure unterscheidet. Das Peptid, Polypeptid oder Protein bindet vorzugsweise an Endothelzellen und/oder an Neuronen des Gehirns oder Rückenmarks.

Im Rahmen der vorliegenden Offenbarung bezeichnet der Begriff "Peptid" eine Verknüpfung von 2-10 Aminosäuren, die über eine peptidische Bindung miteinander verbunden sind. Der Begriff "Polypeptid" bezeichnet eine Verknüpfung von 11-100 Aminosäuren, die über eine peptidische Bindung miteinander verbunden sind. Polypeptide mit mehr als 100 Aminosäuren werden vorliegend als "Protein" bezeichnet.

Die für das Gehirn und das Rückenmark spezifische Peptidsequenz gemäß SEQ ID NO:1 oder einer Variante davon ist erfindungsgemäß Teil eines Kapsid-Proteins eines Virus. Dies bedeutet, dass die für das Gehirn und das Rückenmark spezifische Peptidsequenz als Teil eines Kapsid-Proteins des Virus vorliegt. Um derartige Kapsid-Proteine herzustellen, wird eine entsprechende, für das Peptid kodierende Nukleotidsequenz in die Region des Virus-Genoms kloniert, die für ein Kapsid-Protein des Virus kodiert. Wird die für das Gehirn bzw. das Rückenmark spezifische Sequenz als Teil eines Kapsid-Proteins exprimiert, so kann sie vielfach an der Oberfläche des viralen Vektors präsentiert werden.

Vorzugsweise handelt es sich bei dem Kapsid-Protein um ein solches, das von einem Adeno-assoziierten Virus (AAV) stammt. Bei dem AAV kann es sich um einen beliebigen der im Stand der Technik beschriebenen Serotypen handeln, wobei das Kapsid-Protein vorzugsweise von einem AAV von einem der Serotypen 2, 4, 6, 8 und 9 stammt. Ein Kapsid-Protein eines AAV vom Serotyp 2 ist besonders bevorzugt.

Das Kapsid des AAV-Wildtyps setzt sich aus den Kapsid-Proteinen VP1, VP2 und VP3 zusammen, die überlappend von der cap-Region kodiert werden. Alle drei Proteine weisen einen identischen C-terminalen Bereich auf. Das Kapsid von AAV umfasst etwa 60 Kopien der Proteine VP1, VP2 und VP3, die im Verhältnis 1:1:8 exprimiert werden. Wird eine Nukleotidsequenz, die für eine der vorliegend beschriebenen Peptide mit Spezifität für das Gehirn bzw. das Rückenmark kodiert, C-terminal in den Leserahmen von VP1 kloniert (d.h. in den Bereich, der bei allen drei Proteinen identisch ist), so kann man erwarten, dass theoretisch 60 der spezifischen Peptide der auf der Kapsid-Oberfläche zu finden sind.

Wird ein AAV-Vektor im Sinne der vorliegenden Erfindung modifiziert, so wird die für das Peptid mit Spezifität für das Gehirn kodierende Nukleotidsequenz in die cap-Region am 3'-Ende des Genoms kloniert. Die Sequenz, die für das Peptid mit Spezifität für das Gehirn bzw. Rückenmark kodiert, kann in die genomische Sequenz eines der Kapsid-Proteine VP1, VP2 oder VP3 kloniert werden. Die Kapsid-Proteine von AAV2 sind beispielhaft in SEQ ID NO:7 (VP1), SEQ ID NO:8 (VP2) und SEQ ID NO:9 (VP3) dargestellt.

In einer besonders bevorzugten Ausführungsform der Erfindung wird die Sequenz, die für das Peptid kodiert, welches Spezifität für das Gehirn bzw. Rückenmark aufweist, in den Leserahmen eines VP1-Gens kloniert, vorzugsweise in das in SEQ ID NO:7 gezeigte VP1-Gen von AAV2. Dabei sollte beachtet werden, dass sich durch die Insertierung der klonierten Sequenz kein Wechsel des Leserahmens und kein vorzeitiger Abbruch der Translation ergibt. Dem Fachmann werden die dazu erforderlichen Methoden ohne Weiteres ersichtlich sein.

In allen drei Kapsid-Proteinen von AVV wurden Stellen identifiziert, an denen Peptidsequenzen für das Homing eingefügt werden können [15-20] . So wurde unter anderem das im VP1 von AAV2 vorkommende Arginin in Position 588 (R588) spezifisch für die Insertion eines Peptidliganden vorgeschlagen [21-22]. Diese Aminosäureposition des viralen Kapsids ist offenbar an der Bindung von AAV2 an seinen natürlichen Rezeptor beteiligt. Es wurde vermutet, dass R588 einer von vier Arginin-Resten ist, der die Bindung von AAV2 an seinen natürlichen Rezeptor vermittelt [23-24]. Eine Modifikation in diesem Bereich des Kapsids schwächt den natürlichen Tropismus von AAV2 ab oder beseitigt diesen vollständig.

Demgemäß ist es erfindungsgemäß besonders bevorzugt, dass die Peptidsequenz gemäß SEQ ID NO:1 oder einer Variante davon im Bereich der Aminosäuren 550-600 des VP1-Proteins von AAV2, insbesondere des VP1-Proteins von SEQ ID NO:7, insertiert vorliegt. Noch stärker bevorzugt ist es, dass die Peptidsequenz im Bereich der Aminosäuren 560-600, 570-600, 560-590, 570-590 des VP1-Proteins inseriert vorliegt.

So kann sich die Peptidsequenz gemäß SEQ ID NO:1 oder einer Variante davon z.B. unmittelbar hinter einer der folgenden Aminosäure des VP1-Proteins, insbesondere des Proteins von SEQ ID NO:7, anschließen: 550, 551, 552, 553, 554, 555, 556, 557, 558, 559, 560, 561, 562, 563, 564, 565, 566, 567, 568, 569, 570, 571, 572, 573, 574, 575, 576, 577, 578, 579, 580, 581, 582, 583, 584, 585, 586, 587, 588, 589, 590, 591, 592, 593, 594, 595, 596, 597, 598, 599 oder 600.

Besonders bevorzugt ist es, dass die Peptidsequenz gemäß SEQ ID NO:1 oder einer Variante davon auf die Aminosäure 588 des VP1-Proteins von SEQ ID NO:7 folgt, wie es in den Beispielen gezeigt ist. Es ist dabei möglich, dass zwischen dem Arginin-Rest in Position 588 und der ersten Aminosäure des Peptids oder der Variante davon eine oder mehrere, und insbesondere bis zu 5 (d.h. 1, 2, 3, 4 oder 5) Aminosäuren liegen, die durch die Klonierung bedingt sind. Ebenso können hinter der letzten Aminosäure des Peptids oder der Variante davon eine oder mehrere, insbesondere bis zu 5 (d.h. 1, 2, 3, 4 oder 5) Aminosäuren liegen.

Die oben in Bezug auf VP1 angegebenen Stellen und Bereiche in der Aminosäuresequenz des Kapsid-Proteins gelten analog auch für die Kapsid-Proteine VP2 und VP3 von AAV2. Da sich die drei Kapsid-Proteine VP1, VP2 und VP3 von AAV2 lediglich durch die Länge der N-terminalen Sequenz unterscheiden und demgemäß einen identischen C-terminalen Bereich aufweisen, wird der Fachmann keine Probleme haben, durch Sequenzvergleich die oben für VP1 angegebenen Stellen für die Insertierung des Peptidliganden in den Aminosäuresequenzen von VP1 und VP2 zu identifizieren. So entspricht die Aminosäure R588 in VP1 der Position R451 von VP2 (SEQ ID NO:8) bzw. der Position R386 von VP3 (SEQ ID NO:9).

SEQ ID NO:10 zeigt beispielhaft die Sequenz des VP1-Proteins von AVV2 nach Einbringung der Peptidsequenz von SEQ ID NO:1. Bedingt durch die Klonierung weist das Kapsid-Protein zwei zusätzliche Aminosäuren auf, die in der nativen Sequenz des VP1-Proteins von AVV2 nicht vorkommen. So wird die Peptidsequenz von SEQ ID NO:1 N-terminal von einem Glycin in Position 589 und C-terminal von einem Alanin in Position 597 flankiert. Darüber hinaus ist das Asparagin in Position 587 der nativen Sequenz gegen ein Glutamin ausgetauscht.

In einer besonderen Ausführungsform betrifft die vorliegende Erfindung somit auch ein Kapsid-Protein, das Folgendes umfasst oder daraus besteht:
(a) die Aminosäuresequenz von SEQ ID NO:10;
(b) eine Aminosäuresequenz, die mindestens 80% und vorzugsweise 90, 95 oder 99% Identität zu der Aminosäuresequenz von SEQ ID NO:10 aufweist und darüber hinaus (i) die Sequenz von SEQ ID NO:1 umfasst oder (ii) eine Aminosäuresequenz, die sich von der Aminosäuresequenz von SEQ ID NO:1 durch Modifikation von einer Aminosäure unterscheidet; oder
(c) ein Fragment von einer der in (a) oder (b) definierten Aminosäuresequenzen.

Des Weiteren stellt die vorliegende Erfindung auch eine Nukleinsäure bereit, die für ein wie oben beschriebenes Kapsid-Protein kodiert. Vorzugsweise umfasst die für das Kapsid-Protein kodierende Nukleinsäure die Nukleotidsequenz von SEQ ID NO:11 oder eine davon abgeleitete Nukleotidsequenz mit mindestens 80% Sequenzidentität. Ein Plasmid, das eine solche Nukleinsäure umfasst, wird ebenfalls bereitgestellt.

In einem noch weiteren Aspekt betrifft die Erfindung einen rekombinanten, d.h. einen mittels gentechnischer Verfahren hergestellten, viralen Vektor, der ein Kapsid und mindestens ein darin verpacktes Transgen umfasst, wobei das Kapsid mindestens ein Kapsid-Protein umfasst, welches die Peptidsequenz gemäß SEQ ID NO:1 oder einer Variante davon umfasst. Bei dem rekombinanten viralen Vektor kann es sich um einen rekombinanten AAV-Vektor handeln, z.B. um einen solchen vom Serotyp 2, 4, 6, 8 und 9. AAV-Vektoren vom Serotyp 2 sind besonders bevorzugt.

Die unterschiedlichen AAV-Serotypen unterscheiden sich im Wesentlichen anhand ihres natürlichen Tropismus. So bindet Wildtyp-AAV2 stärker an alveolare Zellen, während AAV5, AAV6 und AAV9 vorwiegend Epithelzellen infizieren. Der Fachmann kann diese natürlichen Unterschiede hinsichtlich der Spezifität der Zellen nutzen, um die durch die oben beschriebenen Peptide vermittelte Spezifität für bestimmte Zellen oder Gewebe weiter zu verstärken. Auf der Nukleinsäureebene sind die verschiedenen AAV-Serotypen stark homolog. So sind z.B. die Serotypen AAV1, AAV2, AAV3 und AAV6 auf der Nukleinsäureebene zu 82% identisch [25].

Das Kapsid der erfindungsgemäßen viralen Vektoren umfasst vorzugsweise ein oder mehrere Transgene. Als Transgen wird vorliegend ein Gen bezeichnet, das mittels gentechnischer Verfahren in das Genom des Vektors eingebracht wurde. Bei dem Transgen (oder den Transgenen) kann es sich um DNA oder RNA handeln. Vorzugsweise handelt es sich um einzelsträngige DNA (ssDNA) oder doppelsträngige DNA (dsDNA), wie beispielsweise genomische DNA oder cDNA. Vorzugsweise handelt es sich bei dem Transgen, welches mit Hilfe des erfindungsgemäßen rekombinanten viralen Vektors transportiert werden soll, um ein humanes Gen. Geeignete Transgene sind z.B. therapeutische Gene, die ein im Patienten dysfunktionales Gen ersetzen sollen. Es kann sich ferner um ein Gen handeln, das in dem entsprechenden Zielgewebe nicht oder nur in unzureichendem Umfang exprimiert wird.

Die erfindungsgemäßen Vektoren können u.a. zur Behandlung der multiplen Sklerose (MS) eingesetzt werden. In diesem Fall kann es sich bei dem Transgen z.B. um ein Gen handeln, das für ein Membran- oder Tight-Junction-Protein kodiert, wie z.B. für ein Claudin und Occludin. Verzugsweise handelt es sich um ein Gen aus der Familie der Claudine handeln, z.B. um das für Claudinl kodierende Gen. Die Überexpression eines solchen Gens könnte hilfreich sein, um die durch die Krankheit geschädigte Blut-Hirn-Schranke "abzudichten".

Ferner kann zur Behandlung der MS auch ein Gen eingesetzt werden, das für einen Chemokin-Antagonisten kodiert. So kann z.B. eine negativ-dominante Mutante des Chemokins CCL2 (aka MCP1) exprimiert werden, die als "CCL2-7ND" bezeichnet wird. CCL2 sorgt dafür, dass Immunzellen zu einem Entzündungsherd gelockt werden, was im Fall der MS zum entzündlichen Abbau der neuronalen Myelinscheide führt. Die dominant-negative Variante "CCL2-7ND" hingegen bildet Dimere und blockiert den Rezeptor CCR2, wodurch die Signalkaskade unterbunden wird und die Immunzellen nicht mehr zu den Neuronen gelockt werden.

Zur Behandlung der Alzheimer-Krankheit kann z.B. das kodierende Gen für Neuraminidasel (NEU1), Neprilysin oder Cholesterol 24-Hydroxylase überexprimiert werden, was zur Abreicherung von Amyloid β-Plaques führen und damit zu einer Verbesserung des Krankheitszustands beitragen kann.

Die Parkinson Krankheit kann durch Vektor-vermittelte Überexpression z.B. des neurotrophischen Faktors der Gliazellen (GDNF), der aromatischen L-Aminosäure-Decarboxylase, der Tyrosynhydroxylase oder der GTP-Cyclohydrolase I behandelt werden, wobei ein positiver Einfluss auf das dopaminergische System zu erwarten wäre. Eine Behandlung der Spinalen Muskelatrophie kann z.B. durch Expression des Proteins SMN (Survival of Motor Neuron) erfolgen.

Für die Therapie einer lysosomalen Speicherkrankheit wäre die Expression einer Glucuronidase, z.B. der β-Glucuronidase, als Transgen mit Hilfe des erfindungsgemäßen Vektors erfolgsversprechend.

In einer weiteren Ausführungsform kodiert das Transgen für ein radioprotektives Protein, wie z.B. für ein radioprotektives Enzym. Eine wesentliche Limitation beim Einsatz der Radiotherapie im Rahmen der Behandlung von Krebs ist die strahleninduzierte Schädigung des Normalgewebes, was häufig eine Verringerung der Strahlendosis, eine Unterbrechung des Therapieregimes, oder sogar einen völligen Verzicht auf diese Therapieform nötig macht. Das Gehirn ist ein besonders strahlenempfindliches Organ, weshalb Primärtumoren dort oft nur eingeschränkt und diffuses Tumorwachstum meistens gar nicht radiotherapeutisch behandelt werden kann. Mit Hilfe der Vektoren der vorliegenden Erfindung kann das gesunde Gewebe, welches das maligne Gewebe umgibt, durch zielgerichtete Expression radioprotektiver Proteine geschützt werden. In einer bevorzugten Ausführungsform handelt es sich bei dem Transgen, welches in das gesunde Gehirngewebe eingeschleust wird, um eine Mangan-Superoxiddismutase (MnSOD), die die Konversion von Superoxidanionen, einem der wesentliche Faktoren der strahleninduzierten Toxizität, zu Wasserstoffperoxid katalysiert. In einer weiteren hier vorgeschlagenen Ausführungsform handelt es sich um die Kinase-Domäne des Ataxia telangiectasia mutant (ATM)-Gens, das zur Reparatur der durch Bestrahlung entstandenen DNA-Schäden beiträgt. In einer weiteren bevorzugten Ausführungsform werden beide Gene mittels der vorliegend beschriebenen Vektoren in den zu behandelnden Patienten eingebracht.

Die viralen Vektoren der vorliegenden Erfindung eignen sich insbesondere zur Verwendung in einem Verfahren zur therapeutischen Behandlung von Erkrankungen des Gehirns und/oder Rückenmarks. Erkrankungen des Gehirns bzw. des Rückenmarks im Sinne der Erfindung umfassen genetisch verursachte Leukodystrophien, wie Adrenoleukodystrophie, Morbus Canavan, Morbus Krabbe, metachromatische Leukodystrophie, Pelizaeus-Merzbacher-Krankheit und Morbus Alexander; neurodegenerative Erkrankungen, wie amyotrophe Lateralsklerose, Alzheimer, Parkinson, Chorea Huntington und Morbus Pick; chronisch-entzündliche Erkrankungen des Zentralnervensystems, wie Multiple Sklerose und Guillain-Barre-Syndrom; und Lysosomen-Speichererkrankungen, wie Ceroid-Lipofuszinose und Morbus Fabry. In einer bevorzugten Ausführungsform werden die viralen Vektoren der vorliegenden Erfindung zur therapeutischen Behandlung von neurodegenerative Erkrankungen, wie Alzheimer, Parkinson oder Chorea Huntington eingesetzt.

In einer noch weiteren Ausführungsform kodiert das Transgen für ein Antitumormittel, wie z.B. ein Tumorsupressor-Protein, oder einen Immunmodulator, wie z.B. ein Zytokin (z.B. Interleukin 1 bis 4, Gamma-Interferon, p53), das selektiv zu einem Gehirntumor oder einen Tumor des Rückenmarks des Patienten transportiert werden soll.

Die Vektoren können ferner auch dazu verwendet werden, um Antisense-RNA, Ribozyme, oder Ähnliches in Zellen und Gewebe des Gehirns bzw. Rückenmarks zu transportieren. Ferner können die erfindungsgemäßen Vektoren auch Transgene umfassen, die für sekretorische Proteine kodieren, die in das Lumen der Mikrovaskulatur des Gehirns oder des Rückenmarks freigesetzt werden sollen. Solche sekretorischen Proteine können beispielsweise entzündungshemmend wirken.

Wie vorliegend verwendet, bezeichnet der Begriff "Subjekt" alle menschlichen oder tierischen Organismen, die durch AAV-Vektoren infiziert werden können. Vorzugsweise handelt es sich bei dem zu behandelnden Subjekt um ein Säugetier, wie z.B. um einen Menschen, einen Primaten, eine Maus oder eine Ratte. In einer bevorzugten Ausführungsform ist das zu behandelnde Subjekt ein Mensch. Nach Transfektion in das Subjekt sorgt der Vektor für die ortsspezifische Expression des Transgens in den Zellen des Gehirns bzw. des Rückenmarks.

Das Transgen kann in dem viralen Vektor in Form einer Expressionskassette vorliegen, die neben der zu exprimierenden Sequenz des Transgens weitere für die Expression erforderliche Elemente umfasst, wie z.B. einen geeigneten Promoter, der nach Infektion der entsprechenden Zellen die Expression des Transgens steuert. Geeignete Promotoren umfassen neben den AAV-Promotoren z.B. den Cytomegalovirus (CMV)-Promoter, den Chicken-Beta-Actin/Cytomegalovirus-Hybridpromoter (CAG), einen Endothelzellen-spezifischen Promoter, wie z.B. den VE-Catherin Promoter, sowie Steroid-Promotoren und Metallothionein-Promotoren. In einer besonders bevorzugten Ausführungsform ist der in den erfindungsgemäßen Vektoren verwendete Promoter ein CAG-Promoter. In einer besonders bevorzugten Ausführungsform umfasst das erfindungsgemäße Transgen einen hirnspezifischen Promoter, der in funktionaler Verbindung mit dem zu exprimierenden Transgen verbunden ist. Auf diese Weise kann die Spezifität der erfindungsgemäßen Vektoren für das Gehirn weiter erhöht werden. Wie hierin verwendet, ist ein hirnspezifischer Promoter ein Promoter, dessen Aktivität in Hirngewebe mindestens 2-fach, 5-fach, 10-fach, 20-fach, 50-fach oder 100-fach höher ist als in einer Zelle, die keine Gehirnzelle ist. Vorzugsweise ist dieser Promoter ein humaner Promoter. Die Expressionskassette kann auch ein Enhancer-Element zur Erhöhung des Expressionslevels des zu exprimierenden exogenen Proteins enthalten. Ferner kann die Expressionskassette Polyadenylierungssequenzen, wie z.B. die SV40-Polyadenylierungssequenzen oder die Polyadenylierungssequenzen des bovinen Wachstumshormons umfassen.

Die erfindungsgemäßen viralen Vektoren können als Bestandteil eines ihrer Kapsid-Proteine vorzugsweise eine Peptidsequenz gemäß SEQ ID NO:1 umfassen. Alternativ können auch Varianten der Aminosäuresequenz von SEQ ID NO:1 verwendet werden, die sich von der Aminosäuresequenz von SEQ ID NO:1 durch Modifikation von einer Aminosäure unterscheiden. Bei der Modifikation kann es sich um einen Austausch, eine Deletion oder eine Insertion von Aminosäuren handeln, solang die Variante die Fähigkeit beibehält, als Teil des Kapsids die spezifische Bindung des Vektors an Rezeptor-Strukturen von Zellen des Gehirns und/oder Rückenmarks zu vermitteln. Die Erfindung erstreckt sich somit z.B. auf Varianten der Sequenz von SEQ ID NO:1, bei denen die C- oder N-terminale Aminosäure verändert wurde. Diese Varianten weisen eine Sequenzidentität von mehr als 85% zu der in SEQ ID NO:1 gezeigten Aminosäuresequenz auf, wenn die Sequenzen mit den Programmen GAP oder BESTFIT miteinander verglichen werden. Diese Computerprogramme zur Bestimmung der Aminosäuresequenzidentität sind im Stand der Technik hinreichend bekannt.

Die Variante der Sequenz gemäß SEQ ID NO:1 kann auf einer Substitution einer Aminosäure beruhen, d.h. eine Aminosäure kann gegen eine andere Aminosäure ausgetauscht sein. Vorzugsweise ist die Substitution, durch die sich die Varianten von der in SEQ ID NO:1 dargestellten Aminosäuresequenz unterscheidet, eine konservative Substitution, d.h. eine Substitution einer Aminosäure durch eine Aminosäure ähnlicher Polarität, die dem Peptid ähnliche funktionelle Eigenschaften verleiht. Vorzugsweise stammt die ausgetauschte Aminosäure aus der gleichen Gruppe von Aminosäuren wie die ersetzende Aminosäure. Beispielsweise kann ein hydrophober Rest durch einen anderen hydrophoben Rest ersetzt werden oder ein polarer Rest durch einen anderen polaren Rest. Funktionell ähnliche Aminosäuren, die im Rahmen einer konservativen Substitution gegeneinander ausgetauscht werden können, umfassen z.B. nicht-polare Aminosäuren wie z.B. Glycin, Valin, Alanin, Isoleucin, Leucin, Methionin, Prolin, Phenylalanin, und Tryptophan. Beispiele von ungeladenen polaren Aminosäuren umfassen Serin, Threonin, Glutamin, Asparagin, Tyrosin, und Cystein. Beispiele von geladenen polaren (sauren) Aminosäuren umfassen Histidin, Arginin und Lysin. Beispiele von geladenen polaren (basischen) Aminosäuren umfassen Asparaginsäure und Glutaminsäure.

Als Varianten der in SEQ ID NO:1 gezeigten Aminosäuresequenz gelten auch solche Aminosäuresequenzen, bei denen eine Aminosäure eingefügt wurde. Solche Insertionen können erfolgen, so lange die resultierende Variante ihre Fähigkeit beibehält, spezifisch an Zellen des Gehirns und/oder des Rückenmarks zu binden. Ferner gelten vorliegend auch solche Proteine als Varianten der in SEQ ID NO:1 gezeigten Aminosäuresequenz, bei denen eine der beiden N-terminalen Aminosäuren des Peptids fehlt. Voraussetzung ist wiederum, dass die entsprechend deletierte Variante spezifisch an Zellen des Gehirns und/oder des Rückenmarks bindet.

Ebenfalls von der Erfindung umfasst sind spezifische Varianten der in SEQ ID NO:1 gezeigten Aminosäuresequenz, die an einer Aminosäure strukturell verändert wurden, wie beispielsweise durch Einführen einer modifizierten Aminosäure. Bei diesen modifizierten Aminosäuren kann es sich erfindungsgemäß um Aminosäuren handeln, die durch Biotinylierung, Phosphorylierung, Glykosylierung, Acetylierung, Verzweigung und/oder Zyklisierung verändert wurden.

Virale Vektoren, deren Kapsid die oben beschriebene Peptidsequenz oder eine wie oben definierte Variante davon umfassen, binden spezifisch Zellen des Gehirns und/oder des Rückenmarks. Wie vorliegend verwendet bedeutet eine "spezifische" Bindung der erfindungsgemäßen Vektoren, dass sich die Vektoren nach systemischer Verabreichung vorwiegend an oder in den Zellen des Gehirns und/oder des Rückenmarks anreichern. Dies bedeutet, dass sich mehr als 50% der ursprünglich verabreichten Vektorgenome im Bereich der Zellen des Gehirns und/oder des Rückenmarks anreichern, während sich weniger als 50% in oder im Bereich von anderen Zellen oder Geweben anreichern (wie z.B. in der Milz oder Leber). Es ist bevorzugt, dass sich mehr als 60%, 70%, 80%, 90%, 95%, oder sogar mehr als 99% der ursprünglich verabreichten Vektorgenome in oder im Bereich der Zellen des Gehirns und/oder des Rückenmarks anreichern. Eine spezifische Bindung der Vektoren kann auch über die Expression des Transgens ermittelt werden. Bei viralen Vektoren, die spezifisch Zellen des Gehirns und/oder des Rückenmarks binden, erfolgt mehr als 50% der gesamten Expression des Transgens in oder im Bereich des Gehirns und/oder des Rückenmarks, während weniger als 50% der Expression in oder im Bereich von anderen Geweben beobachtet werden kann. Es ist allerdings bevorzugt, dass mehr als 60%, 70%, 80%, 90%, 95%, oder sogar mehr als 99% der insgesamt gemessenen Expression des Transgens in oder im Bereich des Gehirns und/oder des Rückenmarks erfolgt.

Der Fachmann wird problemlos in der Lage sein, die spezifische Bindung der erfindungsgemäßen Vektoren an Zellen des Gehirns bzw. des Rückenmarks und die Expression des mit Hilfe der Vektoren eingeschleusten Transgens zu bestimmten. Zu diesem Zweck geeignete Verfahren zur Messung der Spezifität von Transduktion und Expression sind in den nachstehenden Beispielen gezeigt.

Es ist darüber hinaus erfindungsgemäß bevorzugt, dass Vektoren, deren Kapside Varianten der in SEQ ID NO:1 gezeigten Aminosäuresequenz enthalten, mindestens etwa 50% der Bindungsaktivität eines entsprechenden viralen Vektors aufweisen, dessen Kapsid die in SEQ ID NO:1 gezeigte Aminosäuresequenz aufweist. Noch stärker bevorzugt ist es, dass die Varianten etwa 60%, 70%, 80%, 90%, 95%, 96%, 97%, 98% oder 99% der Bindungsaktivität eines entsprechenden viralen Vektors aufweisen, dessen Kapsid die in SEQ ID NO:1 gezeigte Aminosäuresequenz aufweist. Die Bindungsaktivität der Vektoren kann mit Hilfe von in vitro-Assays gemessen werden, wie sie in den vorliegenden Beispielen beschrieben werden.

Vorzugsweise ist die Bindungsaktivität der erfindungsgemäßen Vektoren, wie sie durch Verteilung der Vektorgenome oder Expression des Transgens bestimmt werden kann, für Zellen des Gehirns und/oder des Rückenmarks mindestens 2-fach, 5-fach, 10-fach, 20-fach, 50-fach, 75-fach, 100-fach, 150-fach, 200-fach, 250-fach, 500-fach, 600-fach, 700-fach, 800-fach, 900-fach, 1000-fach, 2000-fach, 5000-fach, oder 10000-fach höher als die Bindungsaktivität für eine Kontroll-Zelle, die keine Zelle des Gehirns oder Rückenmarks ist, Z.B. eine Milz- oder Leber-Zelle).

Die Erfindung betrifft auch eine Zelle, die ein erfindungsgemäßes Kapsid-Protein, eine dafür kodierende Nukleinsäure, ein Plasmid, welches eine solche Nukleinsäure umfasst, oder einen wie oben beschriebenen rekombinanten AAV-Vektor enthält. Es handelt sich vorzugsweise um eine humane Zelle oder Zelllinie.

In einer Ausführungsform wurde eine Zelle z.B. durch ein Biopsieverfahren aus einem humanen Subjekt gewonnen und anschließend mit dem viralen Vektor in einem *ex vivo* Verfahren transfiziert. Die Zelle kann anschließend dem Subjekt reimplantiert oder diesem auf andere Weise wieder zugeführt werden, z.B. durch Transplantation oder Infusion. Die Wahrscheinlichkeit einer Abstoßung von transplantierten Zellen ist geringer, wenn das Subjekt, aus dem die Zelle gewonnen wurde, genetisch dem Subjekt ähnelt, an das die Zelle verabreicht wird. Vorzugsweise handelt es sich daher bei dem Subjekt, dem die transfizierten Zellen zugeführt werden, um dasselbe Subjekt, aus dem zuvor die Zellen gewonnen wurden. Die Zelle ist vorzugsweise eine humane Zelle des Gehirns oder Rückenmarks, insbesondere eine neuronale Zelle, eine Endothelzelle eines Blutgefäßes. Die die zu transfizierende Zelle kann auch eine Stammzelle, wie z.B. eine adulte humane Stammzelle, sein. Es ist erfindungsgemäß besonders bevorzugt, dass es sich bei den zu transfizierenden Zellen um autologen Zellen handelt, die mit dem erfindungsgemäßen viralen Vektor, z.B. dem beschriebenen rekombinanten AAV2-Vektor, *ex vivo* transfiziert wurde. Die Zellen werden vorzugsweise in einem Verfahren zur Behandlung einer Funktionsstörung oder Erkrankung des Gehirns und/oder des Rückenmarks in einem Subjekt verwendet.

In einem weiteren Aspekt betrifft die Erfindung ein Verfahren zur Herstellung eines AAV-Vektors, bei dem ein Plasmid verwendet wird, dass für ein Kapsid-Protein kodiert, welches die Aminosäuresequenz von SEQ ID NO:1 oder einer Variante davon umfasst. Das grundsätzliche Verfahren der Herstellung von rekombinanten AAV-Vektoren, die ein zu exprimierendes Transgen umfassen, ist im Stand der Technik hinreichend beschrieben [28]. HEK293-T Zellen werden mit drei Plasmiden transfiziert. Ein erstes Plasmid enthält das die cap- und rep-Regionen des AAV-Genoms, wobei jedoch die natürlich vorkommenden Inverted Repeats (ITRs) fehlen. Die cap-Region dieses Plasmids enthält ein Gen, das für mindestens ein modifiziertes Kapsid-Protein kodiert, d.h. ein Protein, welches die Peptidsequenz gemäß SEQ ID NO:1 oder einer Variante davon umfasst. Ein zweites Plasmid umfasst eine Transgen-Expressionskassette, die von den entsprechenden ITRs flankiert wird, die das Verpackungssignal darstellen. Die Expressionskassette wird daher im Zuge des Zusammenbaus der Viruspartikel in das Kapsid verpackt. Bei dem dritten Plasmid handelt es sich um ein adenovirales Helferplasmid, auf dem die Helferproteine E1A, E1B, E2A, E4-orf6, VA kodiert sind, die für die AAV-Replikation in den HEK 293-T-Zellen erforderlich sind. Alternativ ist auch möglich, die erfindungsgemäßen AVV-Vektoren in Insektenzellen zu erzeugen. Ein entsprechendes Protokoll ist beispielhaft im unten aufgeführten Beispiel 6 aufgeführt.

Es hat sich überraschend gezeigt, dass die Herstellung der rekombinanten Vektoren deren Spezifität beeinflussen kann. So wurde im Rahmen der vorliegenden Erfindung beobachtet, dass Vektoren, die mit Hilfe des Protokolls von Beispiel 2 in HEK293T-Zellen hergestellt wurden, vorwiegend Endothelzellen der Blutgefäße von Gehirn und Rückenmark transfizierten und nur zu einem geringeren Teil Neuronen des Gehirns oder des Rückenmarks. Anderseits transfizierten die rekombinanten Vektoren, die mit Hilfe des Protokolls von Beispiel 6 in Sf9-Zellen hergestellt wurden, vorwiegend Neuronen und im wesentlich geringeren Umfang Endothelzellen. Diese Beobachtung kann entsprechend genutzt werden, um die Spezifität der Transfektion weiter zu erhöhen.

Bedingungen, welche die Anreicherung und Aufreinigung der erfindungsgemäßen rekombinanten Vektoren erlauben, sind im Stand der Technik bekannt. Die erfindungsgemäßen Vektoren können beispielsweise durch Gelfiltrationsverfahren, z.B. unter Verwendung einer Sepharosematrix, entsalzt und durch anschließende Filtration aufgereinigt werden. Andere Aufreinigungsverfahren können ferner eine Cäsiumchlorid- oder Iodixanol-Gradienten-Ultrazentrifugation umfassen. Die Aufreinigung reduziert potentiell abträgliche Affekte in dem Subjekt, an das die adeno-assoziierten viralen Vektoren verabreicht werden. Der verabreichte Virus ist im Wesentlichen frei von Wildtyp- und replikationskompetenten Virus. Die Reinheit des Virus kann durch geeignete Verfahren, wie PCR-Amplifikation, überprüft werden.

Die Erfindung stellt in einem weiteren Aspekt eine pharmazeutische Zusammensetzung bereit, die einen viralen Vektor der vorliegenden Erfindung, insbesondere einen AAV-Vektor, umfasst. Der virale Vektor wird dabei in einer therapeutisch wirksamen Menge an den Patienten verabreicht, d.h. in einer Menge, die ausreicht, um mindestens ein Symptom der zu behandelnden Funktionsstörung oder Erkrankung des Gehirns und/oder des Rückenmarks in einem erheblichen Maße zu verbessern oder die Progression der Erkrankung zu unterbinden. Eine therapeutisch wirksame Menge des erfindungsgemäßen Vektors ruft eine positive Veränderung in einem der besagten Symptom hervor, d.h. eine Veränderung die den Phänotyp des betroffenen Subjekts an den Phänotyp eines gesunden Subjekts, das nicht unter einer Erkrankung des Gehirns und/oder des Rückenmarks leidet, annähert.

In einer bevorzugten Ausführungsform der Erfindung erfolgt die Verabreichung des viralen Vektors in einer Menge, die zu einer vollständigen oder im Wesentlichen vollständigen Heilung der Funktionsstörung oder Erkrankung des Gehirns und/oder des Rückenmarks führt. Die pharmazeutische Zusammensetzung wird demgemäß eine therapeutisch wirksame Dosis des erfindungsgemäßen Vektors enthalten. Eine therapeutisch wirksame Dosis wird in der Regel für das Subjekt, welches sich der Behandlung unterzieht, nicht toxisch sein.

Die genaue Menge an viralen Vektor, die verabreicht werden muss, um einen therapeutischen Effekt zu erzielen, hängt von mehreren Parametern ab. Faktoren, die relevant für die Menge des zu verabreichenden viralen Vektors sind, umfassen z.B. die Art der Verabreichung des viralen Vektors, die Art und Schwere der Erkrankung, die Krankheitsgeschichte des zu behandelnden Patienten sowie Alter, Gewicht, Größe und Gesundheitszustand des zu behandelnden Patienten. Ferner sind auch das Expressionslevel des Transgens, das benötigt wird um einen therapeutischen Effekt zu erzielen, die Immunantwort des Patienten, sowie die Stabilität des Genproduktes für die zu verabreichende Menge relevant. Eine therapeutisch wirksame Menge des viralen Vektors kann von einem Fachmann ohne Weiteres auf Basis des allgemeinen Fachwissens und der vorliegenden Offenbarung bestimmt werden.

Der virale Vektor wird vorzugsweise in einer Menge verabreicht, die einer Virusdosis im Bereich von 1,0 x 10¹⁰ bis 1,0 x 10¹⁴ vg/kg (Virusgenome pro kg Körpergewicht) entspricht, wobei ein Bereich von 1,0 x 10¹¹ bis 1,0 x 10¹³ vg/kg stärker bevorzugt ist, und ein Bereich von 5,0 x 10¹¹ bis 5,0 x 10¹² vg/kg stärker bevorzugt ist, und ein Bereich von 1,0 x 10¹² bis 5,0 x 10¹² noch stärker bevorzugt ist. Eine Virusdosis von etwa 2,5 x 10¹² vg/kg ist am stärksten bevorzugt. Die zu verabreichende Menge des viralen Vektors, z.B. des erfindungsgemäßen AAV2-Vektors, kann abhängig von der Stärke der Expression des einen oder der mehreren Transgene angepasst werden.

Der virale Vektor der vorliegenden Erfindung, wie z.B. der erfindungsgemäß bevorzugte AVV2-Vektor, kann für verschiedene Arten der Verabreichung formuliert werden, z.B. für die orale Verabreichung als Kapsel, als Flüssigkeit oder Ähnliches. Es ist jedoch bevorzugt, dass der virale Vektor parenteral, vorzugsweise mittels intravenöser Injektion oder intravenöser Infusion verabreicht wird. Die Verabreichung kann beispielsweise mittels intravenöser Infusion, z.B. innerhalb von 60 Minuten, innerhalb von 30 Minuten, oder innerhalb von 15 Minuten erfolgen. Es ist ferner bevorzugt, dass der virale Vektor während einer Operation lokal mittels Injektion in das Gehirn und/oder das Rückenmark verabreicht wird. Zusammensetzungen, die für die Verabreichung mittels Injektion und/oder Infusion geeignet sind, umfassen in der Regel Lösungen und Dispersionen sowie Pulver, aus denen entsprechende Lösungen und Dispersionen hergestellt werden können. Derartige Zusammensetzungen werden den viralen Vektor und mindestens einen geeigneten pharmazeutisch akzeptablen Träger enthalten. Geeignete pharmazeutisch akzeptable Träger für die intravenöse Verabreichung umfassen bakteriostatisches Wasser, Ringer-Lösung, physiologische Salzlösung, Phosphat-gepufferte Salzlösung (PBS) und Cremophor EL™. Sterile Zusammensetzungen für die Injektion und/oder Infusion können hergestellt werden, indem der viralen Vektor in der erforderlichen Menge in einem geeigneten Träger eingebracht und anschließend mittels eines Filters sterilfiltriert wird. Zusammensetzungen für die Verabreichung mittels Injektion oder Infusion sollten nach ihrer Herstellung unter Lagerungsbedingungen über einen längeren Zeitraum stabil bleiben. Die Zusammensetzungen können zu diesem Zweck ein Konservierungsmittel enthalten. Geeignete Konservierungsmittel umfassen Chlorbutanol, Phenol, Ascorbinsäure und Thiomersal. Die Herstellung von entsprechenden Darreichungsformen sowie geeignete Hilfsstoffe sind beispielsweise in "Remington: The Science and Practice of Pharmacy", Lippincott Williams & Wilkins; 21. Auflage (2005) beschrieben.

In noch einem weiteren Aspekt betrifft die Erfindung ein Verfahren zur therapeutischen Behandlung einer Funktionsstörung oder einer Erkrankung des Gehirns und/oder des Rückenmarks, bei dem man einen erfindungsgemäßen viralen Vektor, vorzugsweise einen AAV-Vektor, wie er oben beschrieben ist, an ein Subjekt verabreicht. Der Vektor umfasst ein Kapsid, das mindestens ein Kapsid-Protein umfasst, welches die Peptidsequenz gemäß SEQ ID NO:1 oder einer Variante davon enthält. Der virale Vektor umfasst ferner ein Transgen, z.B. ein therapeutisches Gen, das für die Behandlung der Funktionsstörung oder Erkrankung des Gehirns und/oder des Rückenmarks nützlich ist. Nach Verabreichung an das zu behandelnde Subjekt, vorzugsweise durch systemische Gabe, wie z.B. intravenöse Injektion oder Infusion, sorgt der Vektor für die spezifische Expression des Gens in den Zellen des Gehirns und/oder des Rückenmarks.

### KURZE BESCHREIBUNG DER FIGUREN

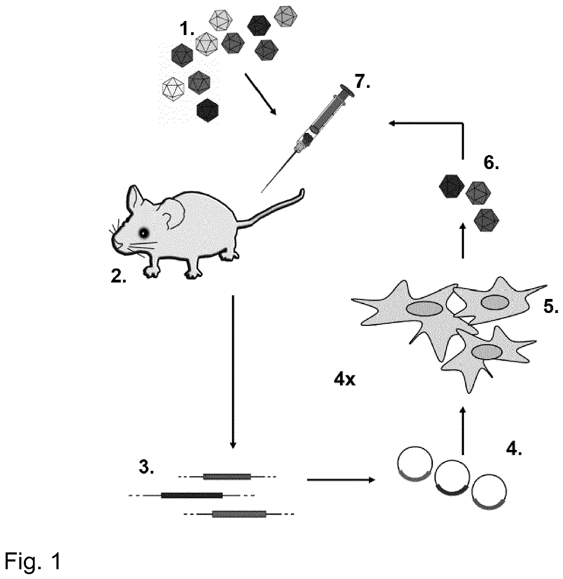

**Figur 1** zeigt das im Rahmen der Erfindung angewendete *in vivo* Selektionsverfahren der AAV-Peptidbank.
   - 1.:: Randomisierte AAV Peptidbibliothek mit ∼1x10⁸ unterschiedlichen Kapsidvarianten;
   - 2.:: Entnahme des Zielorgans, 8 Tage nach Injektion;
   - 3.:: DNS Isolierung und Amplifikation der viralen DNS-Fragmente per Real-Time-PCR;
   - 4.:: Klonierung in Peptidbibliotheks-Plasmide für Sequenzierung und Produktion einer sekundären Peptidbibliothek;
   - 5.:: Co-Transfektion von HEK293T Zellen, Produktion einer sekundären Peptidbibliothek;
   - 6.:: sekundäre AAV Peptidbibliothek für weitere Selektionsrunden. Beinhaltet vorselektierte Kapsidvarianten;
   - 7.:: intravenöse Injektion der Peptidbibliothek in die Maus.

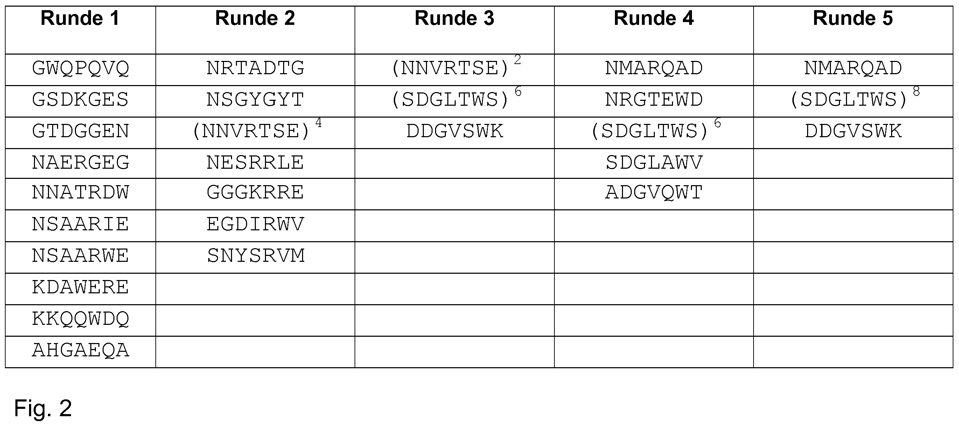

**Figur 2** zeigt die Sequenzen der mittels des Selektionsverfahrens identifizierten, für das Gehirn spezifischen Peptide. Das Peptid mit der in SEQ ID NO:1 gezeigten Sequenz wurde in der vierten Selektionsrunde identifiziert.

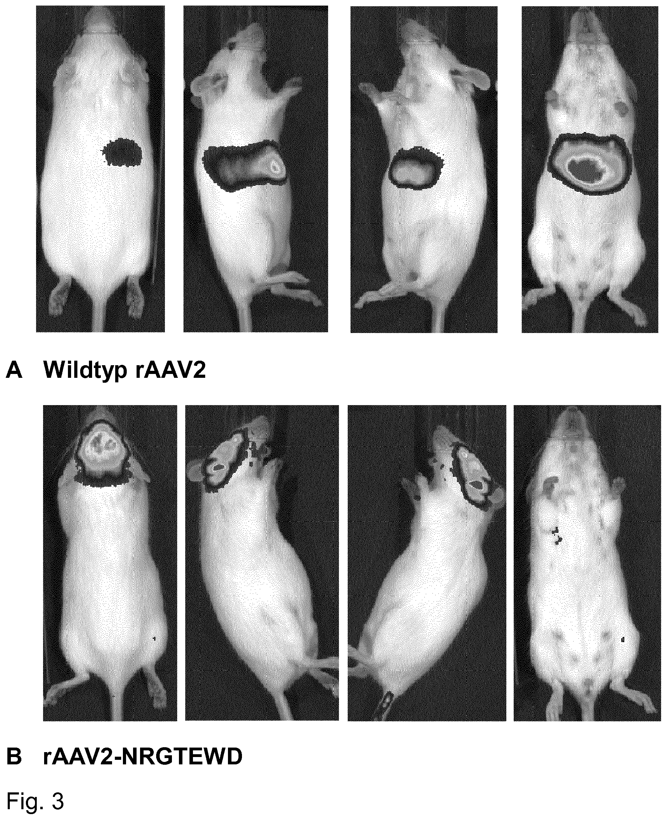

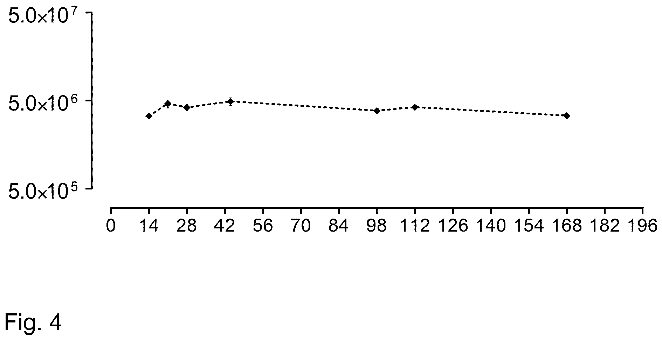

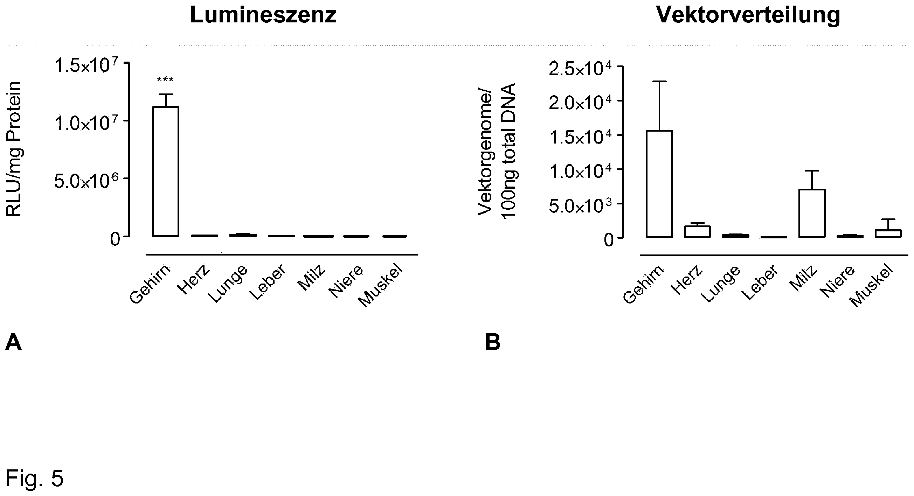

**Figur 3** zeigt die Genexpression nach intravenöser Injektion von rekombinanten AAV-Vektoren in der lebenden Maus. Die Luziferaseexpression wurde 14 Tage nach systemischer Injektion von 5x10¹⁰

## Patentansprüche

1. Kapsid-Protein eines viralen Vektors, das spezifisch an Zellen des Gehirns und/oder des Rückenmarks bindet, **dadurch gekennzeichnet, dass** es folgendes umfasst:
(a) die Aminosäuresequenz von SEQ ID NO:1, oder
(b) eine Aminosäuresequenz, die sich von der Aminosäuresequenz von SEQ ID NO:1 durch Modifikation von einer Aminosäure unterscheidet.

2. Kapsid-Protein nach Anspruch 1, wobei es sich um ein Kapsid-Protein eines Adeno-assoziierten Virus (AAV) handelt.

3. Kapsid-Protein nach Anspruch 2, wobei es sich um ein Kapsid-Protein eines AAV von einem Serotyp ausgewählt aus der Gruppe bestehend aus den 2, 4, 6, 8 und 9 handelt.

4. Kapsid-Protein nach Anspruch 3, wobei es sich um das VP1-Protein eines AAV vom Serotyp 2 handelt.

5. Kapsid-Protein nach einem der Ansprüche 1-4, das folgendes umfasst:
(a) die Aminosäuresequenz von SEQ ID NO:10;
(b) eine Aminosäuresequenz, die mindestens 80% Identität zu der Aminosäuresequenz von SEQ ID NO:10 aufweist und darüber hinaus die Sequenz von SEQ ID NO:1 umfasst oder eine Aminosäuresequenz, die sich von der Aminosäuresequenz von SEQ ID NO:1 durch Modifikation von einer Aminosäure unterscheidet; oder
(c) ein Fragment von einer der in (a) oder (b) definierten Aminosäuresequenzen.

6. Virales Kapsid, das ein Kapsid-Protein nach einem der Ansprüche 1-5 umfasst.

7. Nukleinsäure, die für ein Kapsid-Protein nach einem der Ansprüche 1-5 kodiert.

8. Plasmid, das eine Nukleinsäure nach Anspruch 7 umfasst.

9. Rekombinanter viraler Vektor, der ein virales Kapsid nach Anspruch 6 und ein darin verpacktes Transgen umfasst.

10. Rekombinanter viraler Vektor nach Anspruch 9, wobei es sich um einen rekombinanten AAV-Vektor handelt.

11. Rekombinanter viraler Vektor nach Anspruch 10, wobei es sich um einen AAV-Vektor eines Serotyps ausgewählt aus der Gruppe bestehend aus den Serotypen 2, 4, 6, 8 und 9 handelt.

12. Rekombinanter viraler Vektor nach einem der Ansprüche 9-11, wobei das Transgen für eines der folgenden Proteine kodiert: ein Membran- oder Tight-Junction-Protein, wie z.B. ein Claudin oder Occludin, Neuraminidase, z.B. Neuraminidase 1, Glucuronidase, ein Chemokin-Antagonist, wie z.B. CCL2-7ND, Neurotrophischer Faktor der Gliazellen (GDNF), Neprilysin, Cholesterol 24-Hydroxylase, aromatische L-Aminosäure-Decarboxylase, Tyrosynhydroxylase, GTP-Cyclohydrolase I und Survival of Motor Neuron(SMN)-Protein.

13. Rekombinanter viraler Vektor nach einem der Ansprüche 9-12 zur Verwendung in einem Verfahren zur Behandlung einer Funktionsstörung oder einer Erkrankung des Gehirns und/oder Rückenmarks in einem Subjekt wobei die Erkrankung des Gehirns und/oder Rückenmarks vorzugsweise ausgewählt ist aus der Gruppe bestehend aus genetisch verursachten Leukodystrophien, wie Adrenoleukodystrophie, Morbus Canavan, Morbus Krabbe, metachromatische Leukodystrophie, Pelizaeus-Merzbacher-Krankheit und Morbus Alexander; neurodegenerativen Erkrankungen, wie amyotrophe Lateralsklerose, Alzheimer, Parkinson, Chorea Huntington und Morbus Pick; chronisch-entzündlichen Erkrankungen des Zentralnervensystems, wie Multiple Sklerose und Guillain-Barre-Syndrom; und Lysosomen-Speichererkrankungen, wie Ceroid-Lipofuszinose und Morbus Fabry.

14. Zelle, die ein Kapsid-Protein nach einem der Ansprüche 1-5, ein virales Kapsid nach Anspruch 6, eine Nukleinsäure nach Anspruch 7, ein Plasmid nach Anspruch 8, oder einen rekombinanten viralen Vektor nach einem der Ansprüche 9-12 umfasst.

15. Pharmazeutische Zusammensetzung, die ein Kapsid-Protein nach einem der Ansprüche 1-5, ein virales Kapsid nach Anspruch 6, eine Nukleinsäure nach Anspruch 7, ein Plasmid nach Anspruch 8, oder einen rekombinanten viralen Vektor nach einem der Ansprüche 9-12 umfasst.

16. Kapsid-Protein nach einem der Ansprüche 1-5, virales Kapsid nach Anspruch 6, Nukleinsäure nach Anspruch 7, Plasmid nach Anspruch 8 oder rekombinanter viraler Vektor nach einem der Ansprüche 9-12 zur Verwendung in einem Verfahren zur Behandlung einer Funktionsstörung oder einer Erkrankung des Gehirns und/oder Rückenmarks in einem Subjekt, wobei die Erkrankung des Gehirns und/oder Rückenmarks vorzugsweise ausgewählt ist aus der Gruppe bestehend aus genetisch verursachten Leukodystrophien, wie Adrenoleukodystrophie, Morbus Canavan, Morbus Krabbe, metachromatische Leukodystrophie, Pelizaeus-Merzbacher-Krankheit und Morbus Alexander; neurodegenerativen Erkrankungen, wie amyotrophe Lateralsklerose, Alzheimer, Parkinson, Chorea Huntington und Morbus Pick; chronisch-entzündlichen Erkrankungen des Zentralnervensystems, wie Multiple Sklerose und Guillain-Barre-Syndrom; und Lysosomen-Speichererkrankungen, wie Ceroid-Lipofuszinose und Morbus Fabry.

## Claims

1. Capsid protein of a viral vector which specifically binds to cells of the brain and/or spinal cord, **characterized in that** it comprises the following:
(a) the amino acid sequence of SEQ ID NO:1, or
(b) an amino acid sequence that differs from the amino acid sequence of SEQ ID NO:1 by modification of a single amino acid.

2. Capsid protein according to claim 1, which is a capsid protein of an adeno-associated virus (AAV).

3. Capsid protein according to claim 2, which is a capsid protein of an AAV of a serotype selected from the group consisting of 2, 4, 6, 8 and 9.

4. Capsid protein according to claim 3, which is the VP1 protein of an AAV of serotype 2.

5. Capsid protein according to any of the claims 1-4, which comprises the following:
(a) the amino acid sequence of SEQ ID NO:10;
(b) an amino acid sequence having at least 80% identity to the amino acid sequence of SEQ ID NO:10 and furthermore comprising the sequence of SEQ ID NO:1 or an amino acid sequence that differs from the amino acid sequence of SEQ ID NO:1 by the modification of a single amino acid; or
(c) a fragment of one of the amino acid sequences defined in (a) or (b).

6. Viral capsid comprising a capsid protein according to any of claims 1-5.

7. Nucleic acid encoding a capsid protein according to any of claims 1-5.

8. Plasmid comprising a nucleic acid according to claim 7.

9. Recombinant viral vector comprising a viral capsid according to claim 6 and a transgene packaged therein.

10. Recombinant viral vector according to claim 9, which is a recombinant AAV vector.

11. Recombinant viral vector according to claim 10, which is a AAV vector of a serotype selected from the group consisting of serotypes 2, 4, 6, 8 and 9.

12. Recombinant viral vector according to any of claims 9-11, wherein the transgene encodes one of the following proteins: a membrane or tight junction protein, e.g. a claudin or occludin, neuraminidase, e.g. neuraminidase 1, glucuronidase, a chemokine antagonist, e.g. CCL2-7ND, Glial cell line-derived neurotrophic factor (GDNF), neprilysin, cholesterol 24-hydroxylase, aromatic L-amino acid decarboxylase, tyrosine hydroxylase, GTP cyclohydrolase I and survival of motor neuron (SMN) protein.

13. Recombinant viral vector according to any of claims 9-12 for use in a method of treating a functional disorder or a disease of the brain and/or spinal cord in a subject, wherein said disease of the brain and/or spinal cord is preferably selected from the group consisting of genetically-caused leukodystrophies, such as adrenoleukodystrophy, Canavan disease, Krabbe disease, metachromatic leukodystrophy, Pelizaeus-Merzbacher disease and Alexander disease; neurodegenerative diseases, such as amyotrophic lateral sclerosis, Alzheimer's disease, Parkinson's disease, Huntington's disease and Pick's disease; chronic inflammatory diseases of the central nervous system, such as multiple sclerosis and Guillain-Barre syndrome; and lysosomal storage diseases, such as ceroid lipofuscinosis and Fabry disease.

14. Cell comprising a capsid protein according to any of claims 1-5, a viral capsid according to claim 6, a nucleic acid according to claim 7, a plasmid according to claim 8, or a recombinant viral vector according to any of claims 9-12.

15. Pharmaceutical composition comprising a capsid protein according to any the claims 1-5, a viral capsid according to claim 6, a nucleic acid according to claim 7, a plasmid according to claim 8, or a recombinant viral vector according to any of the claims 9-12.

16. Capsid protein according to any of the claims 1-5, viral capsid according to claim 6, nucleic acid according to claim 7, plasmid according to claim 8, or recombinant viral vector according to any of claims 9-12 for use in a method of treating a functional disorder or a disease of the brain and/or spinal cord in a subject, wherein the disease of the brain and/or spinal cord is preferably selected from the group consisting of genetically-caused leukodystrophies, such as adrenoleukodystrophy, Canavan disease, Krabbe disease, metachromatic leukodystrophy, Pelizaeus-Merzbacher disease and Alexander disease; neurodegenerative diseases, such as amyotrophic lateral sclerosis, Alzheimer's disease, Parkinson's disease, Huntington's disease and Pick's disease; chronic inflammatory diseases of the central nervous system, such as multiple sclerosis and Guillain-Barre syndrome; and lysosomal storage diseases, such as ceroid lipofuscinosis and Fabry disease.

## Revendications

1. Protéine de capside d'un vecteur viral, qui se lie spécifiquement aux cellules du cerveau et/ou de la moelle épinière, **caractérisée en ce qu'**elle comprend ce qui suit :
(a) la séquence d'acides aminés de SEQ ID NO : 1, ou
(b) une séquence d'acides aminés qui diffère de la séquence d'acides aminés de SEQ ID NO : 1 par modification d'un acide aminé.

2. Protéine de capside selon la revendication 1, qui consiste en une protéine de capside d'un virus adéno-associé (AAV).

3. Protéine de capside selon la revendication 2, qui consiste en une protéine de capside d'un AAV d'un sérotype choisi dans l'ensemble constitué par les sérotypes 2, 4, 6, 8 et 9.

4. Protéine de capside selon la revendication 3, qui consiste en la protéine VP1 d'un AAV du sérotype 2.

5. Protéine de capside selon l'une quelconque des revendications 1 à 4, qui comprend ce qui suit :
(a) la séquence d'acides aminés de SEQ ID NO : 10 ;
(b) une séquence d'acides aminés qui présente un degré d'identité d'au moins 80 % avec la séquence d'acides aminés de SEQ ID NO : 10 et en outre comprend la séquence de SEQ ID NO : 1 ou une séquence d'acides aminés qui diffère de la séquence d'acides aminés de SEQ ID NO : 1 par modification d'un acide aminé ; ou
(c) un fragment de l'une des séquences d'acides aminés définies en (a) ou (b).

6. Capside virale, qui comprend une protéine de capside selon l'une quelconque des revendications 1 à 5.

7. Acide nucléique, qui code une protéine de capside selon l'une quelconque des revendications 1 à 5.

8. Plasmide, qui comprend un acide nucléique selon la revendication 7.

9. Vecteur viral recombinant, qui comprend une capside virale selon la revendication 6 et un transgène empaqueté dans celle-ci.

10. Vecteur viral recombinant selon la revendication 9, qui consiste en un vecteur AAV recombinant.

11. Vecteur viral recombinant selon la revendication 10, qui consiste en un vecteur AAV d'un sérotype choisi dans l'ensemble constitué par les sérotypes 2, 4, 6, 8 et 9.

12. Vecteur viral recombinant selon l'une quelconque des revendications 9 à 11, dans lequel le transgène code l'une des protéines suivantes, une protéine membranaire ou de jonction serrée, comme par exemple une claudine ou occludine, une neuraminidase, par exemple la neuraminidase 1, la glucuronidase, un antagoniste de chémokine, comme par exemple CCL2-7ND, le facteur neurotrophique dérivé des cellules gliales (GDNF), la néprilysine, la cholestérol 24-hydroxylase, l'acide aminé L aromatique décarboxylase, la tyrosine hydroxylase, la GTP cyclohydrolase I et la protéine de survie des neurones moteurs (SMN).

13. Vecteur viral recombinant selon l'une quelconque des revendications 9 à 12, destiné à l'utilisation dans un procédé pour le traitement d'un trouble fonctionnel ou d'une maladie du cerveau et/ou de la moelle épinière chez un sujet, la maladie du cerveau et/ou de la moelle épinière étant choisie de préférence dans l'ensemble constitué par les leucodystrophies d'origine génétique, telles que l'adrénoleucodystrophie, la maladie de Canavan, la maladie de Krabbe, la leucodystrophie métachromatique, la maladie de Pelizaeus-Merzbacher et la maladie d'Alexander ; les maladies neurodégénératives, telles que la sclérose amyotrophique latérale, la maladie d'Alzheimer, la maladie de Parkinson, la chorée de Huntington et la maladie de Pick ; les maladies inflammatoires chroniques du système nerveux central, telles que la sclérose en plaques et le syndrome de Guillain-Barré ; et les maladies de stockage lysosomal, telles que la céroïde-lipofuscinose et la maladie de Fabry.

14. Cellule, qui comprend une protéine de capside selon l'une quelconque des revendications 1 à 5, une capside virale selon la revendication 6, un acide nucléique selon la revendication 7, un plasmide selon la revendication 8, ou un vecteur viral recombinant selon l'une quelconque des revendications 9 à 12.

15. Composition pharmaceutique, qui comprend une protéine de capside selon l'une quelconque des revendications 1 à 5, une capside virale selon la revendication 6, un acide nucléique selon la revendication 7, un plasmide selon la revendication 8, ou un vecteur viral recombinant selon l'une quelconque des revendications 9 à 12.

16. Protéine de capside selon l'une quelconque des revendications 1 à 5, capside virale selon la revendication 6, acide nucléique selon la revendication 7, plasmide selon la revendication 8 ou vecteur viral recombinant selon l'une quelconque des revendications 9 à 12, destiné(e) à l'utilisation dans un procédé pour le traitement d'un trouble fonctionnel ou d'une maladie du cerveau et/ou de la moelle épinière chez un sujet, la maladie du cerveau et/ou de la moelle épinière étant choisie de préférence dans l'ensemble constitué par les leucodystrophies d'origine génétique, telles que l'adrénoleucodystrophie, la maladie de Canavan, la maladie de Krabbe, la leucodystrophie métachromatique, la maladie de Pelizaeus-Merzbacher et la maladie d'Alexander ; les maladies neurodégénératives, telles que la sclérose amyotrophique latérale, la maladie d'Alzheimer, la maladie de Parkinson, la chorée de Huntington et la maladie de Pick ; les maladies inflammatoires chroniques du système nerveux central, telles que la sclérose en plaques et le syndrome de Guillain-Barré ; et les maladies de stockage lysosomal, telles que la céroïde-lipofuscinose et la maladie de Fabry.


### SEQUENCE LISTING

<110> Universitätsklinikum Hamburg-Eppendorf
<120> Neue Peptide mit Spezifität für Gehirn und Rückenmark
<130> P 97486
<160> 21
<170> PatentIn version 3.5
<210> 1
   <211> 7
   <212> PRT
   <213> Artificial Sequence
<220>
   <223> brain specific peptide
<400> 1
   Asn Arg Gly Thr Glu Trp Asp
   1               5
<210> 2
   <211> 7
   <212> PRT
   <213> Artificial Sequence
<220>
   <223> brain specific peptide
<400> 2
   Ala Asp Gly Val Gln Trp Thr
   1               5
<210> 3
   <211> 7
   <212> PRT
   <213> Artificial Sequence
<220>
   <223> brain specific peptide
<400> 3
   Asp Asp Gly Val Ser Trp Lys
   1               5
<210> 4
   <211> 7
   <212> PRT
   <213> Artificial Sequence
<220>
   <223> brain specific peptide
<400> 4
   Ser Asp Gly Leu Thr Trp Ser
   1 5
<210> 5
   <211> 7
   <212> PRT
   <213> Artificial Sequence
<220>
   <223> brain specific peptide
<400> 5
   Ser Asp Gly Leu Ala Trp Val
   1 5
<210> 6
   <211> 7
   <212> PRT
   <213> Artificial Sequence
<220>
   <223> consensus sequence
<220>
   <221> misc_feature
   <222> (1)..(1)
   <223> Xaa can be any naturally occurring amino acid
<220>
   <221> misc_feature
   <222> (4)..(5)
   <223> Xaa can be any naturally occurring amino acid
<220>
   <221> misc_feature
   <222> (7)..(7)
   <223> Xaa can be any naturally occurring amino acid
<400> 6
   Xaa Asp Gly Xaa Xaa Trp Xaa
   1 5
<210> 7
   <211> 735
   <212> PRT
   <213> adeno-associated virus 2
<400> 7
<210> 8
   <211> 598
   <212> PRT
   <213> adeno-associated virus 2
<400> 8
   Met Ala Pro Gly Lys Lys Arg Pro Val Glu His Ser Pro Val Glu Pro
   1               5                   10                  15
   Asp Ser Ser Ser Gly Thr Gly Lys Ala Gly Gln Gln Pro Ala Arg Lys
   20 25 30
   Arg Leu Asn Phe Gly Gln Thr Gly Asp Ala Asp Ser Val Pro Asp Pro
   35 40 45
   Gln Pro Leu Gly Gln Pro Pro Ala Ala Pro Ser Gly Leu Gly Thr Asn
   50 55 60
   Thr Met Ala Thr Gly Ser Gly Ala Pro Met Ala Asp Asn Asn Glu Gly
   65 70 75 80
   Ala Asp Gly Val Gly Asn Ser Ser Gly Asn Trp His Cys Asp Ser Thr
   85 90 95
   Trp Met Gly Asp Arg Val Ile Thr Thr Ser Thr Arg Thr Trp Ala Leu
   100 105 110
   Pro Thr Tyr Asn Asn His Leu Tyr Lys Gln Ile Ser Ser Gln Ser Gly
   115 120 125
   Ala Ser Asn Asp Asn His Tyr Phe Gly Tyr Ser Thr Pro Trp Gly Tyr
   130 135 140
   Phe Asp Phe Asn Arg Phe His Cys His Phe Ser Pro Arg Asp Trp Gln
   145 150 155 160
   Arg Leu Ile Asn Asn Asn Trp Gly Phe Arg Pro Lys Arg Leu Asn Phe
   165 170 175
   Lys Leu Phe Asn Ile Gln Val Lys Glu Val Thr Gln Asn Asp Gly Thr
   180 185 190
   Thr Thr Ile Ala Asn Asn Leu Thr Ser Thr Val Gln Val Phe Thr Asp
   195 200 205
   Ser Glu Tyr Gln Leu Pro Tyr Val Leu Gly Ser Ala His Gln Gly Cys
   210 215 220
   Leu Pro Pro Phe Pro Ala Asp Val Phe Met Val Pro Gln Tyr Gly Tyr
   225 230 235 240
   Leu Thr Leu Asn Asn Gly Ser Gln Ala Val Gly Arg Ser Ser Phe Tyr
   245 250 255
   Cys Leu Glu Tyr Phe Pro Ser Gln Met Leu Arg Thr Gly Asn Asn Phe
   260 265 270
   Thr Phe Ser Tyr Thr Phe Glu Asp Val Pro Phe His Ser Ser Tyr Ala
   275 280 285
   His Ser Gln Ser Leu Asp Arg Leu Met Asn Pro Leu Ile Asp Gln Tyr
   290 295 300
   Leu Tyr Tyr Leu Ser Arg Thr Asn Thr Pro Ser Gly Thr Thr Thr Gln
   305 310 315 320
   Ser Arg Leu Gln Phe Ser Gln Ala Gly Ala Ser Asp Ile Arg Asp Gln
   325 330 335
   Ser Arg Asn Trp Leu Pro Gly Pro Cys Tyr Arg Gln Gln Arg Val Ser
   340 345 350
   Lys Thr Ser Ala Asp Asn Asn Asn Ser Glu Tyr Ser Trp Thr Gly Ala
   355 360 365
   Thr Lys Tyr His Leu Asn Gly Arg Asp Ser Leu Val Asn Pro Gly Pro
   370 375 380
   Ala Met Ala Ser His Lys Asp Asp Glu Glu Lys Phe Phe Pro Gln Ser
   385 390 395 400
   Gly Val Leu Ile Phe Gly Lys Gln Gly Ser Glu Lys Thr Asn Val Asp
   405 410 415
   Ile Glu Lys Val Met Ile Thr Asp Glu Glu Glu Ile Arg Thr Thr Asn
   420 425 430
   Pro Val Ala Thr Glu Gln Tyr Gly Ser Val Ser Thr Asn Leu Gln Arg
   435 440 445
   Gly Asn Arg Gln Ala Ala Thr Ala Asp Val Asn Thr Gln Gly Val Leu
   450 455 460
   Pro Gly Met Val Trp Gln Asp Arg Asp Val Tyr Leu Gln Gly Pro Ile
   465 470 475 480
   Trp Ala Lys Ile Pro His Thr Asp Gly His Phe His Pro Ser Pro Leu
   485 490 495
   Met Gly Gly Phe Gly Leu Lys His Pro Pro Pro Gln Ile Leu Ile Lys
   500 505 510
   Asn Thr Pro Val Pro Ala Asn Pro Ser Thr Thr Phe Ser Ala Ala Lys
   515 520 525
   Phe Ala Ser Phe Ile Thr Gln Tyr Ser Thr Gly Gln Val Ser Val Glu
   530 535 540
   Ile Glu Trp Glu Leu Gln Lys Glu Asn Ser Lys Arg Trp Asn Pro Glu
   545 550 555 560
   Ile Gln Tyr Thr Ser Asn Tyr Asn Lys Ser Val Asn Val Asp Phe Thr
   565 570 575
   Val Asp Thr Asn Gly Val Tyr Ser Glu Pro Arg Pro Ile Gly Thr Arg
   580 585 590
   Tyr Leu Thr Arg Asn Leu
   595
<210> 9
   <211> 533
   <212> PRT
   <213> adeno-associated virus 2
<400> 9
   Met Ala Thr Gly Ser Gly Ala Pro Met Ala Asp Asn Asn Glu Gly Ala
   1 5 10 15
   Asp Gly Val Gly Asn Ser Ser Gly Asn Trp His Cys Asp Ser Thr Trp
   20 25 30
   Met Gly Asp Arg Val Ile Thr Thr Ser Thr Arg Thr Trp Ala Leu Pro
   35 40 45
   Thr Tyr Asn Asn His Leu Tyr Lys Gln Ile Ser Ser Gln Ser Gly Ala
   50 55 60
   Ser Asn Asp Asn His Tyr Phe Gly Tyr Ser Thr Pro Trp Gly Tyr Phe
   65 70 75 80
   Asp Phe Asn Arg Phe His Cys His Phe Ser Pro Arg Asp Trp Gln Arg
   85 90 95
   Leu Ile Asn Asn Asn Trp Gly Phe Arg Pro Lys Arg Leu Asn Phe Lys
   100 105 110
   Leu Phe Asn Ile Gln Val Lys Glu Val Thr Gln Asn Asp Gly Thr Thr
   115 120 125
   Thr Ile Ala Asn Asn Leu Thr Ser Thr Val Gln Val Phe Thr Asp Ser
   130 135 140
   Glu Tyr Gln Leu Pro Tyr Val Leu Gly Ser Ala His Gln Gly Cys Leu
   145 150 155 160
   Pro Pro Phe Pro Ala Asp Val Phe Met Val Pro Gln Tyr Gly Tyr Leu
   165 170 175
   Thr Leu Asn Asn Gly Ser Gln Ala Val Gly Arg Ser Ser Phe Tyr Cys
   180 185 190
   Leu Glu Tyr Phe Pro Ser Gln Met Leu Arg Thr Gly Asn Asn Phe Thr
   195 200 205
   Phe Ser Tyr Thr Phe Glu Asp Val Pro Phe His Ser Ser Tyr Ala His
   210 215 220
   Ser Gln Ser Leu Asp Arg Leu Met Asn Pro Leu Ile Asp Gln Tyr Leu
   225 230 235 240
   Tyr Tyr Leu Ser Arg Thr Asn Thr Pro Ser Gly Thr Thr Thr Gln Ser
   245 250 255
   Arg Leu Gln Phe Ser Gln Ala Gly Ala Ser Asp Ile Arg Asp Gln Ser
   260 265 270
   Arg Asn Trp Leu Pro Gly Pro Cys Tyr Arg Gln Gln Arg Val Ser Lys
   275 280 285
   Thr Ser Ala Asp Asn Asn Asn Ser Glu Tyr Ser Trp Thr Gly Ala Thr
   290 295 300
   Lys Tyr His Leu Asn Gly Arg Asp Ser Leu Val Asn Pro Gly Pro Ala
   305 310 315 320
   Met Ala Ser His Lys Asp Asp Glu Glu Lys Phe Phe Pro Gln Ser Gly
   325 330 335
   Val Leu Ile Phe Gly Lys Gln Gly Ser Glu Lys Thr Asn Val Asp Ile
   340 345 350
   Glu Lys Val Met Ile Thr Asp Glu Glu Glu Ile Arg Thr Thr Asn Pro
   355 360 365
   Val Ala Thr Glu Gln Tyr Gly Ser Val Ser Thr Asn Leu Gln Arg Gly
   370 375 380
   Asn Arg Gln Ala Ala Thr Ala Asp Val Asn Thr Gln Gly Val Leu Pro
   385 390 395 400
   Gly Met Val Trp Gln Asp Arg Asp Val Tyr Leu Gln Gly Pro Ile Trp
                   405                 410                 415
   Ala Lys Ile Pro His Thr Asp Gly His Phe His Pro Ser Pro Leu Met
               420                 425                 430
   Gly Gly Phe Gly Leu Lys His Pro Pro Pro Gln Ile Leu Ile Lys Asn
           435                 440                 445
   Thr Pro Val Pro Ala Asn Pro Ser Thr Thr Phe Ser Ala Ala Lys Phe
       450                 455                 460
   Ala Ser Phe Ile Thr Gln Tyr Ser Thr Gly Gln Val Ser Val Glu Ile
   465                 470                 475                 480
   Glu Trp Glu Leu Gln Lys Glu Asn Ser Lys Arg Trp Asn Pro Glu Ile
                   485                 490                 495
   Gln Tyr Thr Ser Asn Tyr Asn Lys Ser Val Asn Val Asp Phe Thr Val
               500                 505                 510
   Asp Thr Asn Gly Val Tyr Ser Glu Pro Arg Pro Ile Gly Thr Arg Tyr
           515                 520                 525
   Leu Thr Arg Asn Leu
       530
<210> 10
   <211> 744
   <212> PRT
   <213> adeno-associated virus 2
<400> 10
   Met Ala Ala Asp Gly Tyr Leu Pro Asp Trp Leu Glu Asp Thr Leu Ser
   1               5                   10                  15
   Glu Gly Ile Arg Gln Trp Trp Lys Leu Lys Pro Gly Pro Pro Pro Pro
               20                  25                  30
   Lys Pro Ala Glu Arg His Lys Asp Asp Ser Arg Gly Leu Val Leu Pro
           35                  40                  45
   Gly Tyr Lys Tyr Leu Gly Pro Phe Asn Gly Leu Asp Lys Gly Glu Pro
       50                  55                  60
   Val Asn Glu Ala Asp Ala Ala Ala Leu Glu His Asp Lys Ala Tyr Asp
   65                  70                  75                  80
   Arg Gln Leu Asp Ser Gly Asp Asn Pro Tyr Leu Lys Tyr Asn His Ala
                   85                  90                  95
   Asp Ala Glu Phe Gln Glu Arg Leu Lys Glu Asp Thr Ser Phe Gly Gly
               100                 105                 110
   Asn Leu Gly Arg Ala Val Phe Gln Ala Lys Lys Arg Val Leu Glu Pro
           115                 120                 125
   Leu Gly Leu Val Glu Glu Pro Val Lys Thr Ala Pro Gly Lys Lys Arg
       130                 135                 140
   Pro Val Glu His Ser Pro Val Glu Pro Asp Ser Ser Ser Gly Thr Gly
   145                 150                 155                 160
   Lys Ala Gly Gln Gln Pro Ala Arg Lys Arg Leu Asn Phe Gly Gln Thr
                   165                 170                 175
   Gly Asp Ala Asp Ser Val Pro Asp Pro Gln Pro Leu Gly Gln Pro Pro
               180                 185                 190
   Ala Ala Pro Ser Gly Leu Gly Thr Asn Thr Met Ala Thr Gly Ser Gly
           195                 200                 205
   Ala Pro Met Ala Asp Asn Asn Glu Gly Ala Asp Gly Val Gly Asn Ser
       210                 215                 220
   Ser Gly Asn Trp His Cys Asp Ser Thr Trp Met Gly Asp Arg Val Ile
   225                 230                 235                 240
   Thr Thr Ser Thr Arg Thr Trp Ala Leu Pro Thr Tyr Asn Asn His Leu
                   245                 250                 255
   Tyr Lys Gln Ile Ser Ser Gln Ser Gly Ala Ser Asn Asp Asn His Tyr
               260                 265                 270
   Phe Gly Tyr Ser Thr Pro Trp Gly Tyr Phe Asp Phe Asn Arg Phe His
           275                 280                 285
   Cys His Phe Ser Pro Arg Asp Trp Gln Arg Leu Ile Asn Asn Asn Trp
       290                 295                 300
   Gly Phe Arg Pro Lys Arg Leu Asn Phe Lys Leu Phe Asn Ile Gln Val
   305                 310                 315                 320
   Lys Glu Val Thr Gln Asn Asp Gly Thr Thr Thr Ile Ala Asn Asn Leu
   325 330 335
   Thr Ser Thr Val Gln Val Phe Thr Asp Ser Glu Tyr Gln Leu Pro Tyr
   340 345 350
   Val Leu Gly Ser Ala His Gln Gly Cys Leu Pro Pro Phe Pro Ala Asp
   355 360 365
   Val Phe Met Val Pro Gln Tyr Gly Tyr Leu Thr Leu Asn Asn Gly Ser
   370 375 380
   Gln Ala Val Gly Arg Ser Ser Phe Tyr Cys Leu Glu Tyr Phe Pro Ser
   385 390 395 400
   Gln Met Leu Arg Thr Gly Asn Asn Phe Thr Phe Ser Tyr Thr Phe Glu
   405 410 415
   Asp Val Pro Phe His Ser Ser Tyr Ala His Ser Gln Ser Leu Asp Arg
   420 425 430
   Leu Met Asn Pro Leu Ile Asp Gln Tyr Leu Tyr Tyr Leu Ser Arg Thr
   435 440 445
   Asn Thr Pro Ser Gly Thr Thr Thr Gln Ser Arg Leu Gln Phe Ser Gln
   450 455 460
   Ala Gly Ala Ser Asp Ile Arg Asp Gln Ser Arg Asn Trp Leu Pro Gly
   465 470 475 480
   Pro Cys Tyr Arg Gln Gln Arg Val Ser Lys Thr Ser Ala Asp Asn Asn
   485 490 495
   Asn Ser Glu Tyr Ser Trp Thr Gly Ala Thr Lys Tyr His Leu Asn Gly
   500 505 510
   Arg Asp Ser Leu Val Asn Pro Gly Pro Ala Met Ala Ser His Lys Asp
   515 520 525
   Asp Glu Glu Lys Phe Phe Pro Gln Ser Gly Val Leu Ile Phe Gly Lys
   530 535 540
   Gln Gly Ser Glu Lys Thr Asn Val Asp Ile Glu Lys Val Met Ile Thr
   545 550 555 560
   Asp Glu Glu Glu Ile Arg Thr Thr Asn Pro Val Ala Thr Glu Gln Tyr
   565 570 575
   Gly Ser Val Ser Thr Asn Leu Gln Arg Gly Gln Arg Gly Asn Arg Gly
               580                 585                 590
   Thr Glu Trp Asp Ala Gln Ala Ala Thr Ala Asp Val Asn Thr Gln Gly
           595                 600                 605
   Val Leu Pro Gly Met Val Trp Gln Asp Arg Asp Val Tyr Leu Gln Gly
       610                 615                 620
   Pro Ile Trp Ala Lys Ile Pro His Thr Asp Gly His Phe His Pro Ser
   625                 630                 635                 640
   Pro Leu Met Gly Gly Phe Gly Leu Lys His Pro Pro Pro Gln Ile Leu
                   645                 650                 655
   Ile Lys Asn Thr Pro Val Pro Ala Asn Pro Ser Thr Thr Phe Ser Ala
               660                 665                 670
   Ala Lys Phe Ala Ser Phe Ile Thr Gln Tyr Ser Thr Gly Gln Val Ser
           675                 680                 685
   Val Glu Ile Glu Trp Glu Leu Gln Lys Glu Asn Ser Lys Arg Trp Asn
       690                 695                 700
   Pro Glu Ile Gln Tyr Thr Ser Asn Tyr Asn Lys Ser Val Asn Val Asp
   705                 710                 715                 720
   Phe Thr Val Asp Thr Asn Gly Val Tyr Ser Glu Pro Arg Pro Ile Gly
                   725                 730                 735
   Thr Arg Tyr Leu Thr Arg Asn Leu
               740
<210> 11
   <211> 8359
   <212> DNA
   <213> adeno-associated virus 2
<400> 11
   catcatcaat aatatacctt attttggatt gaagccaata tgataatgag ggggtggagt      60
   ttgtgacgtg gcgcggggcg tgggaacggg gcgggtgacg tagtagctct agaggtcctg     120
   tattagaggt cacgtgagtg ttttgcgaca ttttgcgaca ccatgtggtc acgctgggta     180
   tttaagcccg agtgagcacg cagggtctcc attttgaagc gggaggtttg aacgcgcagc     240
   cgccatgccg gggttttacg agattgtgat taaggtcccc agcgaccttg acgagcatct      300
   gcccggcatt tctgacagct ttgtgaactg ggtggccgag aaggaatggg agttgccgcc     360
   agattctgac atggatctga atctgattga gcaggcaccc ctgaccgtgg ccgagaagct     420
   gcagcgcgac tttctgacgg aatggcgccg tgtgagtaag gcaccggagg cccttttctt 480
   tgtgcaattt gagaagggag agagctactt ccacatgcac gtgctcgtgg aaaccaccgg 540
   ggtgaaatcc atggttttgg gacgtttcct gagtcagatt cgcgaaaaac tgattcagag 600
   aatttaccgc gggatcgagc cgactttgcc aaactggttc gcggtcacaa agaccagaaa 660
   tggcgccgga ggcgggaaca aggtggtgga tgagtgctac atccccaatt acttgctccc 720
   caaaacccag cctgagctcc agtgggcgtg gactaatatg gaacagtatt taagcgcctg 780
   tttgaatctc acggagcgta aacggttggt ggcgcagcat ctgacgcacg tgtcgcagac 840
   gcaggagcag aacaaagaga atcagaatcc caattctgat gcgccggtga tcagatcaaa 900
   aacttcagcc aggtacatgg agctggtcgg gtggctcgtg gacaagggga ttacctcgga 960
   gaagcagtgg atccaggagg accaggcctc atacatctcc ttcaatgcgg cctccaactc 1020
   gcggtcccaa atcaaggctg ccttggacaa tgcgggaaag attatgagcc tgactaaaac 1080
   cgcccccgac tacctggtgg gccagcagcc cgtggaggac atttccagca atcggattta 1140
   taaaattttg gaactaaacg ggtacgatcc ccaatatgcg gcttccgtct ttctgggatg 1200
   ggccacgaaa aagttcggca agaggaacac catctggctg tttgggcctg caactaccgg 1260
   gaagaccaac atcgcggagg ccatagccca cactgtgccc ttctacgggt gcgtaaactg 1320
   gaccaatgag aactttccct tcaacgactg tgtcgacaag atggtgatct ggtgggagga 1380
   ggggaagatg accgccaagg tcgtggagtc ggccaaagcc attctcggag gaagcaaggt 1440
   gcgcgtggac cagaaatgca agtcctcggc ccagatagac ccgactcccg tgatcgtcac 1500
   ctccaacacc aacatgtgcg ccgtgattga cgggaactca acgaccttcg aacaccagca 1560
   gccgttgcaa gaccggatgt tcaaatttga actcacccgc cgtctggatc atgactttgg 1620
   gaaggtcacc aagcaggaag tcaaagactt tttccggtgg gcaaaggatc acgtggttga 1680
   ggtggagcat gaattctacg tcaaaaaggg tggagccaag aaaagacccg cccccagtga 1740
   cgcagatata agtgagccca aacgggtgcg cgagtcagtt gcgcagccat cgacgtcaga 1800
   cgcggaagct tcgatcaact acgcagacag gtaccaaaac aaatgttctc gtcacgtggg 1860
   catgaatctg atgctgtttc cctgcagaca atgcgagaga atgaatcaga attcaaatat 1920
   ctgcttcact cacggacaga aagactgttt agagtgcttt cccgtgtcag aatctcaacc 1980
   cgtttctgtc gtcaaaaagg cgtatcagaa actgtgctac attcatcata tcatgggaaa 2040
   ggtgccagac gcttgcactg cctgcgatct ggtcaatgtg gatttggatg actgcatctt 2100
   tgaacaataa atgatttaaa tcaggtatgg ctgccgatgg ttatcttcca gattggctcg 2160
   aggacactct ctctgaagga ataagacagt ggtggaagct caaacctggc ccaccaccac 2220
   caaagcccgc agagcggcat aaggacgaca gcaggggtct tgtgcttcct gggtacaagt 2280
   acctcggacc cttcaacgga ctcgacaagg gagagccggt caacgaggca gacgccgcgg 2340
   ccctcgagca cgacaaagcc tacgaccggc agctcgacag cggagacaac ccgtacctca 2400
   agtacaacca cgccgacgcg gagtttcagg agcgccttaa agaagatacg tcttttgggg 2460
   gcaacctcgg acgagcagtc ttccaggcga aaaagagggt tcttgaacct ctgggcctgg 2520
   ttgaggaacc tgttaagacg gctccgggaa aaaagaggcc ggtagagcac tctcctgtgg 2580
   agccagactc ctcctcggga accggaaagg cgggccagca gcctgcaaga aaaagattga 2640
   attttggtca gactggagac gcagactcag tacctgaccc ccagcctctc ggacagccac 2700
   cagcagcccc ctctggtctg ggaactaata cgatggctac aggcagtggc gcaccaatgg 2760
   cagacaataa cgagggcgcc gacggagtgg gtaattcctc gggaaattgg cattgcgatt 2820
   ccacatggat gggcgacaga gtcatcacca ccagcacccg aacctgggcc ctgcccacct 2880
   acaacaacca cctctacaaa caaatttcca gccaatcagg agcctcgaac gacaatcact 2940
   actttggcta cagcacccct tgggggtatt ttgacttcaa cagattccac tgccactttt 3000
   caccacgtga ctggcaaaga ctcatcaaca acaactgggg attccgaccc aagagactca 3060
   acttcaagct ctttaacatt caagtcaaag aggtcacgca gaatgacggt acgacgacga 3120
   ttgccaataa ccttaccagc acggttcagg tgtttactga ctcggagtac cagctcccgt 3180
   acgtcctcgg ctcggcgcat caaggatgcc tcccgccgtt cccagcagac gtcttcatgg 3240
   tgccacagta tggatacctc accctgaaca acgggagtca ggcagtagga cgctcttcat 3300
   tttactgcct ggagtacttt ccttctcaga tgctgcgtac cggaaacaac tttaccttca 3360
   gctacacttt tgaggacgtt cctttccaca gcagctacgc tcacagccag agtctggacc 3420
   gtctcatgaa tcctctcatc gaccagtacc tgtattactt gagcagaaca aacactccaa 3480
   gtggaaccac cacgcagtca aggcttcagt tttctcaggc cggagcgagt gacattcggg 3540
   accagtctag gaactggctt cctggaccct gttaccgcca gcagcgagta tcaaagacat 3600
   ctgcggataa caacaacagt gaatactcgt ggactggagc taccaagtac cacctcaatg 3660
   gcagagactc tctggtgaat ccgggcccgg ccatggcaag ccacaaggac gatgaagaaa 3720
   agtttttccc tcagagcggg gttctcatct ttgggaagca aggctcagag aaaacaaatg 3780
   tggacattga aaaggtcatg attacagacg aagaggaaat caggacaacc aatcccgtgg 3840
   ctacggagca gtatggttct gtatctacca acctccagag aggccagaga ggcaatcggg 3900
   ggactgagtg ggatgcccag gcggccaccg cagatgtcaa cacacaaggc gttcttccag 3960
   gcatggtctg gcaggacaga gatgtgtacc ttcaggggcc catctgggca aagattccac 4020
   acacggacgg acattttcac ccctctcccc tcatgggtgg attcggactt aaacaccctc 4080
   ctccacagat tctcatcaag aacaccccgg tacctgcgaa tccttcgacc accttcagtg 4140
   cggcaaagtt tgcttccttc atcacacagt actccacggg acaggtcagc gtggagatcg 4200
   agtgggagct gcagaaggaa aacagcaaac gctggaatcc cgaaattcag tacacttcca 4260
   actacaacaa gtctgttaat gtggacttta ctgtggacac taatggcgtg tattcagagc 4320
   ctcgccccat tggcaccaga tacctgactc gtaatctgta attgcttgtt aatcaataaa 4380
   ccgtttaatt cgtttcagtt gaactttggt ctctgcgtat ttctttctta tctagtttcc 4440
   atgctctaga ggtcctgtat tagaggtcac gtgagtgttt tgcgacattt tgcgacacca 4500
   tgtggtcacg ctgggtattt aagcccgagt gagcacgcag ggtctccatt ttgaagcggg 4560
   aggtttgaac gcgcagccac cacggcgggg ttttacgaga ttgtgattaa ggtccccagc 4620
   gaccttgacg agcatctgcc cggcatttct gacagctttg tgaactgggt ggccgagaag 4680
   gaatgggagt tgccgccaga ttctgacatg gatctgaatc tgattgagca ggcacccctg 4740
   accgtggccg agaagctgca tcgctggcgt aatagcgaag aggcccgcac cgatcgccct 4800
   tcccaacagt tgcgcagcct gaatggcgaa tggcgattcc gttgcaatgg ctggcggtaa 4860
   tattgttctg gatattacca gcaaggccga tagtttgagt tcttctactc aggcaagtga 4920
   tgttattact aatcaaagaa gtattgcgac aacggttaat ttgcgtgatg gacagactct 4980
   tttactcggt ggcctcactg attataaaaa cacttctcag gattctggcg taccgttcct 5040
   gtctaaaatc cctttaatcg gcctcctgtt tagctcccgc tctgattcta acgaggaaag 5100
   cacgttatac gtgctcgtca aagcaaccat agtacgcgcc ctgtagcggc gcattaagcg 5160
   cggcgggtgt ggtggttacg cgcagcgtga ccgctacact tgccagcgcc ctagcgcccg 5220
   ctcctttcgc tttcttccct tcctttctcg ccacgttcgc cggctttccc cgtcaagctc 5280
   taaatcgggg gctcccttta gggttccgat ttagtgcttt acggcacctc gaccccaaaa 5340
   aacttgatta gggtgatggt tcacgtagtg ggccatcgcc ctgatagacg gtttttcgcc 5400
   ctttgacgtt ggagtccacg ttctttaata gtggactctt gttccaaact ggaacaacac 5460
   tcaaccctat ctcggtctat tcttttgatt tataagggat tttgccgatt tcggcctatt 5520
   ggttaaaaaa tgagctgatt taacaaaaat ttaacgcgaa ttttaacaaa atattaacgt 5580
   ttacaattta aatatttgct tatacaatct tcctgttttt ggggcttttc tgattatcaa 5640
   ccggggtaca tatgattgac atgctagttt tacgattacc gttcatcgat tctcttgttt 5700
   gctccagact ctcaggcaat gacctgatag cctttgtaga gacctctcaa aaatagctac 5760
   cctctccggc atgaatttat cagctagaac ggttgaatat catattgatg gtgatttgac 5820
   tgtctccggc ctttctcacc cgtttgaatc tttacctaca cattactcag gcattgcatt 5880
   taaaatatat gagggttcta aaaattttta tccttgcgtt gaaataaagg cttctcccgc 5940
   aaaagtatta cagggtcata atgtttttgg tacaaccgat ttagctttat gctctgaggc 6000
   tttattgctt aattttgcta attctttgcc ttgcctgtat gatttattgg atgttggaat 6060
   tcctgatgcg gtattttctc cttacgcatc tgtgcggtat ttcacaccgc atatggtgca 6120
   ctctcagtac aatctgctct gatgccgcat agttaagcca gccccgacac ccgccaacac 6180
   ccgctgacgc gccctgacgg gcttgtctgc tcccggcatc cgcttacaga caagctgtga 6240
   ccgtctccgg gagctgcatg tgtcagaggt tttcaccgtc atcaccgaaa cgcgcgagac 6300
   gaaagggcct cgtgatacgc ctatttttat aggttaatgt catgataata atggtttctt 6360
   agacgtcagg tggcactttt cggggaaatg tgcgcggaac ccctatttgt ttatttttct 6420
   aaatacattc aaatatgtat ccgctcatga gacaataacc ctgataaatg cttcaataat 6480
   attgaaaaag gaagagtatg agtattcaac atttccgtgt cgcccttatt cccttttttg 6540
   cggcattttg ccttcctgtt tttgctcacc cagaaacgct ggtgaaagta aaagatgctg 6600
   aagatcagtt gggtgcacga gtgggttaca tcgaactgga tctcaacagc ggtaagatcc 6660
   ttgagagttt tcgccccgaa gaacgttttc caatgatgag cacttttaaa gttctgctat 6720
   gtggcgcggt attatcccgt attgacgccg ggcaagagca actcggtcgc cgcatacact 6780
   attctcagaa tgacttggtt gagtactcac cagtcacaga aaagcatctt acggatggca 6840
   tgacagtaag agaattatgc agtgctgcca taaccatgag tgataacact gcggccaact 6900
   tacttctgac aacgatcgga ggaccgaagg agctaaccgc ttttttgcac aacatggggg 6960
   atcatgtaac tcgccttgat cgttgggaac cggagctgaa tgaagccata ccaaacgacg 7020
   agcgtgacac cacgatgcct gtagcaatgg caacaacgtt gcgcaaacta ttaactggcg 7080
   aactacttac tctagcttcc cggcaacaat taatagactg gatggaggcg gataaagttg 7140
   caggaccact tctgcgctcg gcccttccgg ctggctggtt tattgctgat aaatctggag 7200
   ccggtgagcg tgggtctcgc ggtatcattg cagcactggg gccagatggt aagccctccc 7260
   gtatcgtagt tatctacacg acggggagtc aggcaactat ggatgaacga aatagacaga 7320
   tcgctgagat aggtgcctca ctgattaagc attggtaact gtcagaccaa gtttactcat 7380
   atatacttta gattgattta aaacttcatt tttaatttaa aaggatctag gtgaagatcc 7440
   tttttgataa tctcatgacc aaaatccctt aacgtgagtt ttcgttccac tgagcgtcag 7500
   accccgtaga aaagatcaaa ggatcttctt gagatccttt ttttctgcgc gtaatctgct 7560
   gcttgcaaac aaaaaaacca ccgctaccag cggtggtttg tttgccggat caagagctac 7620
   caactctttt tccgaaggta actggcttca gcagagcgca gataccaaat actgtccttc 7680
   tagtgtagcc gtagttaggc caccacttca agaactctgt agcaccgcct acatacctcg 7740
   ctctgctaat cctgttacca gtggctgctg ccagtggcga taagtcgtgt cttaccgggt 7800
   tggactcaag acgatagtta ccggataagg cgcagcggtc gggctgaacg gggggttcgt 7860
   gcacacagcc cagcttggag cgaacgacct acaccgaact gagataccta cagcgtgagc 7920
   tatgagaaag cgccacgctt cccgaaggga gaaaggcgga caggtatccg gtaagcggca 7980
   gggtcggaac aggagagcgc acgagggagc ttccaggggg aaacgcctgg tatctttata 8040
   gtcctgtcgg gtttcgccac ctctgacttg agcgtcgatt tttgtgatgc tcgtcagggg 8100
   ggcggagcct atggaaaaac gccagcaacg cggccttttt acggttcctg gccttttgct 8160
   ggccttttgc tcacatgttc tttcctgcgt tatcccctga ttctgtggat aaccgtatta 8220
   ccgcctttga gtgagctgat accgctcgcc gcagccgaac gaccgagcgc agcgagtcag 8280
   tgagcgagga agcggaagag cgcccaatac gcaaaccgcc tctccccgcg cgttggccga 8340
   ttcattaatg cagaattcc 8359
<210> 12
   <211> 56
   <212> DNA
   <213> Artificial Sequence
<220>
   <223> PCR Primer
<220>
   <221> misc_feature
   <222> (18)..(38)
   <223> n is a, c, g, or t
<400> 12
   cagtcggcca gagaggcnnn nnnnnnnnnn nnnnnnnngc ccaggcggct gacgag 56
<210> 13
   <211> 16
   <212> DNA
   <213> Artificial Sequence
<220>
   <223> PCR Primer
<400> 13
   ctcgtcagcc gcctgg 16
<210> 14
   <211> 26
   <212> DNA
   <213> Artificial Sequence
<220>
   <223> PCR Primer
<400> 14
   gcagtatggt tctgtatcta ccaacc 26
<210> 15
   <211> 22
   <212> DNA
   <213> Artificial Sequence
<220>
   <223> PCR Primer
<400> 15
   gcctggaaga acgccttgtg tg 22
<210> 16
   <211> 22
   <212> DNA
   <213> Artificial Sequence
<220>
   <223> PCR Primer
<400> 16
   atggcaagcc acaaggacga tg 22
<210> 17
   <211> 22
   <212> DNA
   <213> Artificial Sequence
<220>
   <223> PCR Primer
<400> 17
   cgtggagtac tgtgtgatga ag 22
<210> 18
   <211> 23
   <212> DNA
   <213> Artificial Sequence
<220>
   <223> PCR Primer
<400> 18
   ggttctcatc tttgggaagc aag 23
<210> 19
   <211> 21
   <212> DNA
   <213> Artificial Sequence
<220>
   <223> PCR Primer
<400> 19
   tgatgagaat ctgtggagga g 21
<210> 20
   <211> 19
   <212> DNA
   <213> Artificial Sequence
<220>
   <223> PCR Primer
<400> 20
   ggcggagttg ttacgacat 19
<210> 21
   <211> 20
   <212> DNA
   <213> Artificial Sequence
<220>
   <223> PCR Primer
<400> 21
   gggactttcc ctacttggca 20